# EUROPEAN PATENT APPLICATION

(11) **EP 1 632 491 A1**
(43) Date of publication of application: **08.03.2006**
(21) Application number: 04020535.3
(22) Date of filing: 30.08.2004
(51) Int. Cl.: C07D 409/12, C07D 209/16, C07D 513/04, A61K 31/4045

(54) **Substituted indole compounds and their use as 5-HT6 receptor modulators**

(71) Applicant: LABORATORIOS DEL DR. ESTEVE, S.A., 08041 Barcelona (ES)
(72) Inventor: Merce Vidal, Ramon., E-Barcelona (ES)
(74) Representative: Davila Baz, Angel

(57) **Abstract**

The present invention relates to substituted indole compound of general formula I, 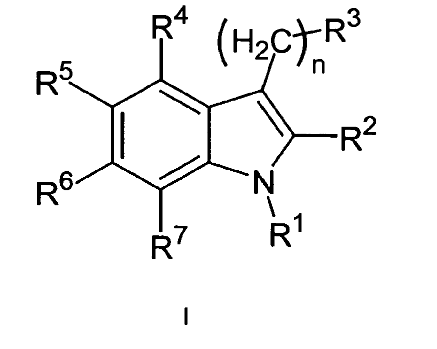 a process for their preparation, medicaments comprising substituted indole compounds as well as the use of substituted indole compounds for the preparation of medicaments, which are suitable e.g. for the prophylaxis and/or treatment of disorders or diseases that are at least partially mediated via 5-HT₆ receptors.

## Description

The present invention relates to substituted indole compounds of general formula 1, 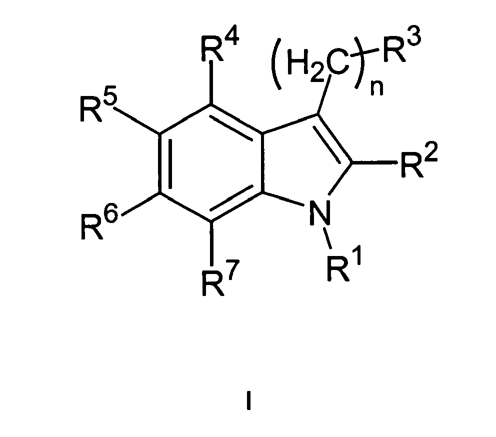 a process for their preparation, medicaments comprising said substituted indole compounds as well as the use of said substituted indole compounds for the preparation of medicaments, which are particularly suitable for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993, 193, 268] and in humans [R. Kohen, et al., J. Neurochem., 1996, 66, 47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol. , 1998, 125, 1562; D.C. Rogers et al., Br. J. Pharmacol. Suppl., 1999, 127, 22P; A. Bourson et al., J. Pharmacol. Exp. Ther. , 1995, 274, 173; A.J. Sleight, et al., Behav. Brain Res. , 1996, 73, 245; T.A. Branchek et al., Annu. Rev. Pharmacol. Toxicol. , 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther. , 1994, 268, 1403; C.E. Glatt et al., Mol. Med. , 1995, 1, 398; F.J. Mosma,et al., Mol. Pharmacol. , 1993, 43, 320; T. Shinkai et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst et al., Br. J. Pharmacol. , 2000, 130, 1597; C. Gerard et al., Brain Research , 1997, 746, 207; M.R. Pranzatelli, Drugs of Today , 1997, 33, 379].

Recently, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

Therefore an object of the present invention was to provide compounds that are particularly suitable as active ingredients in medicaments, especially in medicaments for the prophylaxis and/or treatment of disorders or diseases related to 5-HT₆ receptors such as food intake related disorders.

Surprisingly, it has been found that the substituted indole compounds of general formula I given below show good to excellent affinity for 5-HT₆-receptors. These compounds are therefore particularly suitable as pharmacologically active agents in a medicament for the prophylaxis and/or treatment of disorders or diseases related to 5-HT₆-receptors such as food intake related disorders like obesity.

Thus, in one aspect the present invention relates to substituted indole compounds of general formula I 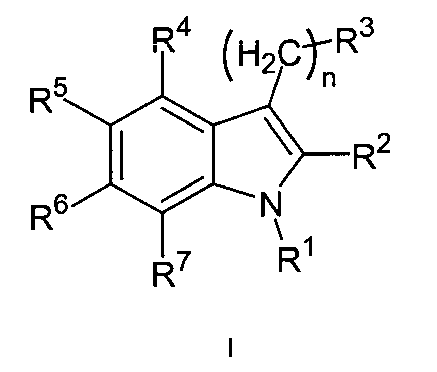
wherein
n is 0, 1, 2, 3 or 4,
R¹ represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; an optionally at least mono-substituted aryl or heteroaryl radical, which may be bonded via a linear or branched alkylene group; a ―C(=O)-R⁸ moiety; or a ―S(=O)₂-R⁹ moiety,
R² represents a hydrogen atom; ―NO₂; -NH₂; -SH; -OH; -CN; -C(=O)-OH; -O-R¹⁰; -S-R¹¹; -C(=O)-OR¹²; a halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R³ represents a saturated or unsaturated, optionally at least mono-substituted, optionally at least one heteroatom as a ring member containing cycloaliphatic radical, which may be condensed with an optionally at least mono-substituted mono- or polycyclic ring system; or a ―NR¹³R¹⁴ moiety,
R⁴, R⁵, R⁶ and R⁷, identical or different, each represent a hydrogen atom; ―NO₂; - NH₂; -SH; -OH; -CN; -C(=O)-OH; -C(=O)-H; -S(=O)₂-OH; -C(=O)-NH₂; -S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷; - NR¹⁸ R¹⁹; -C(=O)-NHR²⁰, -C(=O)-NR²¹R²²; -S(=O)₂-NHR²³, -S(=O)₂-NR²⁴R²⁵; -O-C(=O)-R²⁶; -NH-C(=O)-R²⁷; -NR²⁸-C(=O)-R²⁹; NH-C(=O)-O-R³⁰; NR³¹-C(=O)-O-R³²; - S(=O)₂-O-R³³; a halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group
with the proviso that at least one of the substituents R⁴, R⁵, R⁶ and R⁷ represents an -N(R¹⁵)-S(=O)₂-R¹⁶ moiety,
R⁸ represents a hydrogen atom or a linear or branched, saturated or unsaturated, optionally at least mono-substituted aliphatic radical,
R¹², R¹⁷, R¹⁸, R¹⁹, R²⁰, R²¹, R²², R²³, R²⁴, R²⁵, R²⁶, R²⁷, R²⁸, R²⁹, R³⁰, R³¹, R³² and R³³, independent from one another, each represent a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene, alkenylene or alkinylene group,
R⁹ represents an optionally at least mono-substituted aryl or heteroaryl radical, which may be bonded via a linear or branched optionally at least mono-substituted alkylene, alkenylene or alkinylene group and/or which may be condensed with an optionally at least mono-substituted mono- or polycyclic ring system,
R¹⁰ and R¹¹, independent from one another, each represent a linear or branched, saturated or unsaturated, optionally at least mono-substituted aliphatic radical; or an optionally at least mono-substituted aryl or heteroaryl radical, which may be bonded via a linear or branched alkylene group,
R¹³ and R¹⁴, independent from one another, each represent a hydrogen atom; or a linear or branched, saturated or unsaturated, optionally at least mono-substituted aliphatic radical,
or
R¹³ and R¹⁴ together with the bridging nitrogen form an optionally at least mono-substituted, saturated, unsaturated or aromatic heterocyclic ring which may contain at least one further heteroatom as a ring member and/or which may be condensed with an optionally at least mono-substituted mono- or polycyclic ring system,
R¹⁵ represents a hydrogen atom; a linear or branched, saturated or unsaturated, optionally at least mono-substituted aliphatic radical or a ―S(=O)₂-R¹⁶ moiety,
and
R¹⁶ represents a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched optionally at least mono-substituted alkylene, alkenylene or alkinylene group and/or which may be condensed with an optionally at least mono-substituted mono- or polycyclic ring system; or an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

A mono- or polycyclic ring system according to the present invention ― if not defined otherwise - means a mono- or polycyclic hydrocarbon ring system that may be saturated, unsaturated or aromatic. If the ring system is polycyclic, e.g. bicyclic, each of its different rings may show a different degree of saturation, i.e. it may be saturated, unsaturated or aromatic. Optionally each of the rings of the mono- or polycyclic ring system may contain one or more, preferably 1, 2 or 3, heteroatom(s) as ring member(s), which may be identical or different and which can preferably be selected from the group consisting of N, O and S. Preferably the polycyclic ring system may comprise two rings that are condensed. The rings of the mono- or polycyclic ring-sytem are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

Such a mono- or polycyclic ring system may ― if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅₋alkyl, oxo (=O), thia (=S), -C(=O)-O-C₁₋₅-alkyl, -O-(C=O)-C₁₋₅-alkyl, F, Cl, Br, l, -CN, - CF₃, -OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -CO-N(C₁₋₅-alkyl)₂, -S(=O)₂-C₁₋₅₋alkyl, -S(=O)₂-phenyl, cyclopropyl, cyclobutyl, cyclopentyl, cyclohexyl, phenyl, phenoxy and benzyl, whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, iso-propyl, methoxy, ethoxy, F, CI, Br, -CN, -CF₃, -OCF₃, - SCF₃, -OH, -SH, -NH₂ and NO₂.

If any of the substituents represents or comprises a (hetero)cycloaliphatic radical [(cycloaliphatic) group], said (hetero)cycloaliphatic radical may ― if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅₋alkyl, -S-C₁₋₅-alkyl, -C(=O)-O-C₁₋₅-alkyl, -O-(C=O)-C₁₋₅-alkyl, oxo (=O), thia (=S), F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -CO-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl, whereby in each occurence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, - SCF₃, -OH, -SH, -NH₂ and NO₂.

If any of the substituents represents or comprises a cycloaliphatic radical, which contains one or more, preferably 1, 2 or 3 heteroatom(s) as ring member(s), unless defined otherwise, each of these heteroatom(s) may preferably be selected from the group consisting of of N, 0 and S.

Suitable (hetero)cycloaliphatic radicals, which may be unsubstituted or at least mono-substituted, include cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl or a moiety selected from the group consisting of

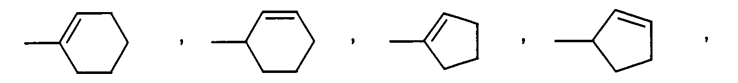

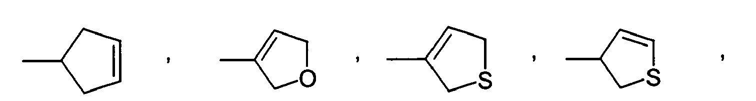

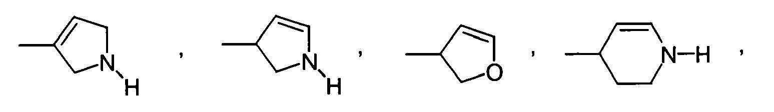

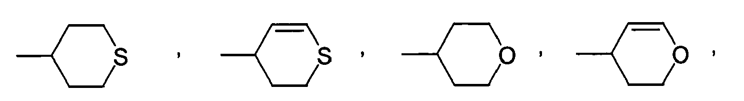

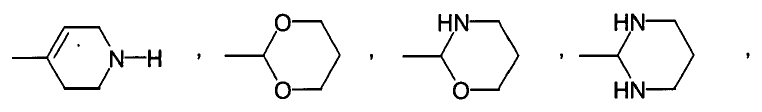

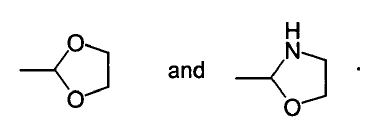

Those skilled in the art understand that the afore mentioned cyclic moities, which contain an NH group may also be substituted in this position, i.e. the hydrogen atom may be exchanged for another substituent.

If any of the substituents represents or comprises a (hetero)cycloaliphatic radical which may be condensed with an optionally at least mono-substituted mono- or polycyclic ring system, said (hetero)cycloaliphatic radical may preferably be selected from the group consisting of

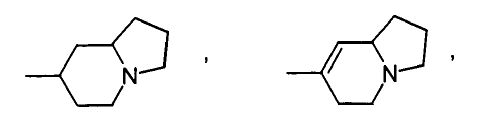

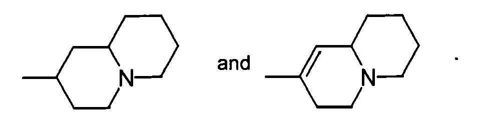

If any of the substituents represents or comprises an aryl radical (aryl group), including a phenyl or naphthyl group, said aryl radical may ― if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅₋alkyl, -C(=O)-O-C₁₋₅-alkyl, -O-(C=O)-C₁₋₅-alkyl, F, Cl, Br, l, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and NO₂.

Preferred aryl radicals, which may optionally be at least mono-substituted, are phenyl and naphthyl.

If any of the substituents represents or comprises a heteroaryl radical (heteroaryl group), said heteroaryl radical may ― if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, - C(=O)-O-C₁₋₅-alkyl, -O-(C=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -CO-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl, whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and NO₂.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2 or 3 heteroatom(s).

Suitable heteroaryl radicals, which may optionally be at least mono-substituted, may preferably be selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl.

If any of the substituents represents or comprises an aryl or heteroaryl radical which may be condensed with an optionally at least mono-substituted mono- or polycyclic ring system, said aryl or heteroaryl radicals may preferably be selected from the group consisting of indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl.

If at least two of the substituents together with the bridging nitrogen atom form a saturated or unsaturated heterocyclic ring which may be condensed with an optionally at least mono-substituted mono- or polycyclic ring system, said heterocyclic rings may preferably be selected from the group consisting of 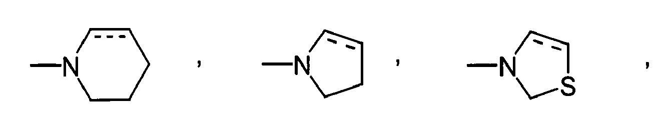 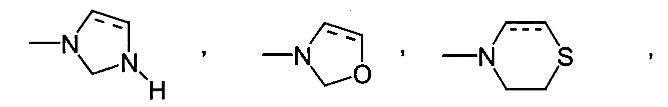 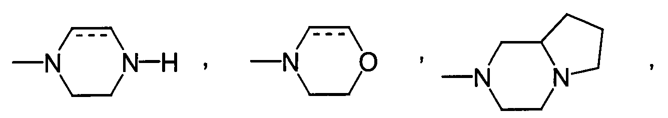 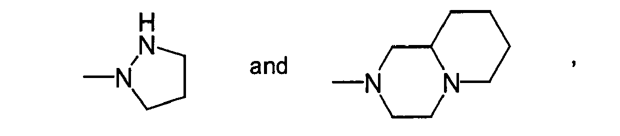
wherein - If present - the dotted line represents an optional chemical bond.

Those skilled in the art understand that the afore mentioned cyclic moities, which contain an NH group may also be substituted in this position, i.e. the hydrogen atom may be exchanged for another substituent.

Said heterocyclic rings may ― if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, - C(=O)-O-C₁₋₅-alkyl, -O-(C=O)-C₁₋₅-alkyl, oxo (=O), thia (=S), F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -CO-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl, whereby in each occurence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, - NH₂ and NO₂,

If any of the substituents represents a saturated or unsaturated aliphatic radical (aliphatic group), i.e. an alkyl radical, an alkenyl radical or an alkinyl radical, said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl) and -N(C₁₋₅-alkyl)₂ , whereby in each occurence C₁₋₅₋alkyl may be linear or branched. An alkenyl radical comprises at least one carbon-carbon double bond, an alkinyl radical comprises at least one carbon-carbon triple bond.

Suitable alkyl radicals, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl) and -N(C₁₋₅-alkyl)₂ , whereby in each occurence C₁₋₅₋alkyl may be linear or branched. An alkenylene group comprises at least one carbon-carbon double bond, an alkinylene group comprises at least one carbon-carbon triple bond.

Suitable alkylene groups include ―(CH₂)-, ―(CH₂)₂-, ―(CH₂)₃-,―(CH₂)₄-,―(CH₂)₅ and ― (CH₂)₆-, suitable alkenylene groups include ―CH=CH-, ―CH₂-CH=CH- and -CH=CH-CH₂- and suitable alkinylene groups include -C≡C- , -CH₂-C≡C- and -C≡C-CH₂-.

In another aspect the present invention relates to substituted indole compounds of general formula I given above,
wherein
n is 0, 1, 2, 3 or 4,
R¹ represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; an optionally at least mono-substituted 5- to 14-membered aryl or heteroaryl radical, which may be bonded via a linear or branched C₁₋₆ alkylene group; a ―C(=O)-R⁸ moiety; or a ―S(=O)₂-R⁹ moiety,
R² represents a hydrogen atom; ―NO₂; -NH₂; -SH; -OH; -CN; -C(=O)-OH; -O-R¹⁰; -S-R¹¹; -C(=O)-OR¹²; a halogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R³ represents a saturated or unsaturated, optionally at least mono-substituted, optionally at least one heteroatom as a ring member containing 3- to 9-membered cycloaliphatic radical, which may be condensed with an optionally at least mono-substituted mono- or polycyclic ring system; or a ―NR¹³R¹⁴ moiety,
R⁴, R⁵, R⁶ and R⁷, identical or different, each represent a hydrogen atom; ―NO₂; - NH₂; -SH; -OH; -CN; -C(=O)-OH; -C(=O)-H; -S(=O)₂-OH; -C(=O)-NH₂; -S(=O)₂-NH₂; - C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, - NR¹⁸R¹⁹; -C(=O)-NHR²⁰, -C(=O)-NR²¹R²²; -S(=O)₂-NHR²³, -S(=O)₂-NR²⁴R²⁵; -O-C(=O)-R²⁶; -NH-C(=O)-R²⁷; -NR²⁸-C(=O)-R²⁹; NH-C(=O)-O-R³⁰; NR³¹-C(=O)-O-R³²; - S(=O)₂-O-R³³; a halogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
with the proviso that at least one of the substituents R⁴, R⁵, R⁶ and R⁷ represents an - N(R¹⁵)-S(=O)₂-R¹⁶ moiety,
R⁸ represents a hydrogen atom or a linear or branched, saturated or unsaturated, optionally at least mono-substituted C₁₋₁₀ aliphatic radical,
R¹², R¹⁷, R¹⁸, R¹⁹, R²⁰, R²¹, R²², R²³, R²⁴, R²⁵, R²⁶, R²⁷, R²⁸, R²⁹, R³⁰, R³¹, R³² and R³³, independent from one another, each represent a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene, C₂₋₆ alkenylene or C₂₋₆ alkinylene group,
R⁹ represents an optionally at least mono-substituted 5- to 14-membered aryl or heteroaryl radical, which may be bonded via a linear or branched optionally at least mono-substituted C₁₋₆ alkylene, C₂₋₆ alkenylene or C₂₋₆ alkinylene group and/or which may be condensed with an optionally at least mono-substituted mono- or polycyclic ring system,
R¹⁰ and R¹¹, independent from one another, each represent a linear or branched, saturated or unsaturated, optionally at least mono-substituted C₁₋₁₀ aliphatic radical; or an optionally at least mono-substituted 5- to 14-membered aryl or heteroaryl radical, which may be bonded via a linear or branched C₁₋₆ alkylene group,
R¹³ and R¹⁴, independent from one another, each represent a hydrogen atom; or a linear or branched, saturated or unsaturated, optionally at least mono-substituted C₁₋₁₀ aliphatic radical,
or
R¹³ and R¹⁴ together with the bridging nitrogen form an optionally at least mono-substituted, saturated, unsaturated or aromatic 3- to 9-membered heterocyclic ring which may contain at least one further heteroatom as a ring member and/or which may be condensed with an optionally at least mono-substituted mono- or polycyclic ring system,
R¹⁵ represents a hydrogen atom; a linear or branched, saturated or unsaturated, optionally at least mono-substituted C₁₋₁₀ aliphatic radical or a ―S(=O)₂-R¹⁶ moiety,
and
R¹⁶ represents a linear or branched, saturated or unsaturated, optionally at least mono-substituted C₁₋₁₀ aliphatic radical; a saturated or unsaturated, optionally at least mono-substituted, optionally at least one heteroatom as a ring member containing 3-to 9-membered cycloaliphatic radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system; or an optionally at least mono-substituted 5- to 14-membered aryl or heteroaryl radical, which may be bonded via a linear or branched, optionally at least mono-substituted C₁₋₆ alkylene, C₂₋₆ alkenylene or C₂₋₆ alkinylene group and/or which may be condensed with an optionally at least mono-substituted mono- or polycyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferably the following proviso may apply to the definition of the substituents R⁴, R⁵ , R⁶ and R⁷ given herein, namely that at least one of the substituents R⁴, R⁵, R⁶ and R⁷ represents an -N(R¹⁵)-S(=O)₂-R¹⁶ moiety and at least one of the other substituents of R⁴, R⁵, R⁶ and R⁷ does not represent a hydrogen atom.

Also preferably the following proviso may apply to the definition of the substituents R⁴, R⁵, R⁶ and R⁷ given herein, namely that if R⁵ represents an -N(R¹⁵)-S(=O)₂-R¹⁶ moiety at least one, preferably one, of the substituents R², R⁴, R⁶ and R⁷ does not represent hydrogen.

Preferred are compounds of general formula I given above, wherein R⁴ represents an -N(R¹⁵)-S(=O)₂-R¹⁶ moiety and n and R¹ to R³ and R⁵to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Preferred are also compounds of general formula I given above, wherein R⁵ represents an -N(R¹⁵)-S(=O)₂-R¹⁶ moiety and n and R¹ to R⁴ and R⁶ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Preferred are also compounds of general formula I given above, wherein R⁶ represents an -N(R¹⁵)-S(=O)₂-R¹⁶ moiety and n and R¹ to R⁵ and R⁷ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Preferred are also compounds of general formula I given above, wherein R⁷ represents an -N(R¹⁵)-S(=O)₂-R¹⁶ moiety and n and R¹ to R⁶ and R⁸ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are compounds of general formula I given above, wherein R¹ represents a hydrogen atom; a linear or branched, optionally at least mono-substituted C₁₋₁₀ alkyl radical; a linear or branched, optionally at least mono-substituted C₂₋₆ alkenyl radical; a linear or branched, optionally at least mono-substituted C₂₋₆ alkinyl radical; a saturated or unsaturated, optionally at least mono-substituted 3- to 9-membered cycloaliphatic radical, which may be bonded via a linear or branched C₁₋₆ alkylene group and/or which may contain 1, 2 or 3 heteroatom(s) independently selected from the group consisting of nitrogen, oxygen and sulphur as ring member(s); an optionally at least mono-substituted 5- to 10-membered aryl or heteroaryl radical, which may be bonded via a linear or branched C₁₋₆ alkylene group and wherein the heteroaryl radical contains 1, 2 or 3 heteroatom(s) independently selected from the group consisting of nitrogen, oxygen and sulphur as ring member(s); a ―C(=O)-R⁸ moiety; or a ―S(=O)₂-R⁹ moiety;
preferably R¹ represents a hydrogen atom; an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH₂)_{1,2} or ₃- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; a ―C(=O)-R⁸ moiety; or a ―S(=O)₂-R⁹ moiety;
more preferably R¹ represents a hydrogen atom;
and n and R² to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore, such substituted indole compounds of general formula I given above are preferred, wherein R² represents a hydrogen atom; ―NO₂; -NH₂; -SH; -OH; -CN; - C(=O)-OH; -CF₃; -O-R¹⁰; -S-R¹¹; -C(=O)-OR¹²; F; Cl; Br; I; a linear or branched, unsubstituted C₁₋₁₀ alkyl radical; a linear or branched unsubstituted C₂₋₆ alkenyl radical; a linear or branched, unsubstituted C₂₋₆alkinyl radical; a saturated or unsaturated, optionally at least mono-substituted 3- to 9-membered cycloaliphatic radical, which may be bonded via a linear or branched C₁₋₆ alkylene group and/or which may contain 1, 2 or 3 heteroatom(s) independently selected from the group consisting of nitrogen, oxygen and sulphur as ring member(s); or an optionally at least mono-substituted aryl or heteroaryl radical, which may be bonded via a linear or branched C₁₋₆ alkylene group and wherein the heteroaryl radical contains 1, 2 or 3 heteroatom(s) independently selected from the group consisting of nitrogen, oxygen and sulphur as ring member(s);
preferably R² represents a hydrogen atom; ―NO₂; -NH₂; -SH; -OH; -CN; -C(=O)-OH; -CF₃; ; -O-R¹⁰; -S-R¹¹; -C(=O)-OR¹²_{;} F; Cl; Br; I; an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
more preferably R² represents a hydrogen atom;
and n, R¹ and R³ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are substituted indole compounds of general formula I given above, wherein R³ represents a saturated or unsaturated, optionally at least mono-substituted 3- to 9-membered cycloaliphatic radical which may contain 1, 2 or 3 heteroatom(s) independently selected from the group consisting of nitrogen, oxygen and sulphur as ring member(s) and which may be condensed with an optionally at least mono-substituted mono- or bicyclic ring system,
whereby the rings of the ring system are 5- 6- or 7-membered and may contain 1, 2 or 3 heteroatom(s) independently selected from the group consisting of nitrogen, oxygen and sulphur;
or R³ represents a ―NR¹³R¹⁴ moiety;
preferably R³ represents a moiety selected from the group consisting of 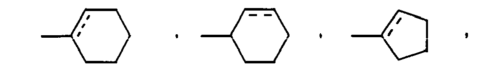 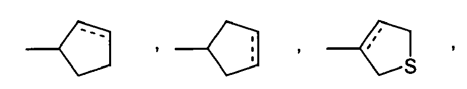 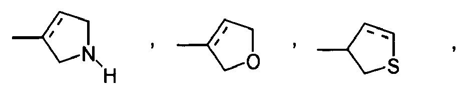 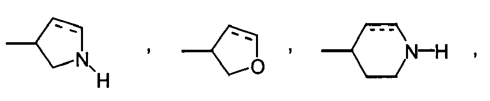 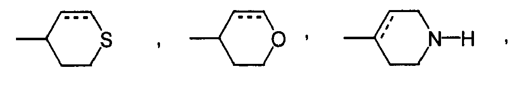 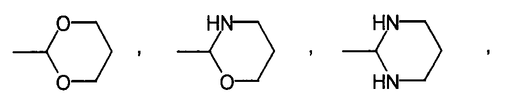 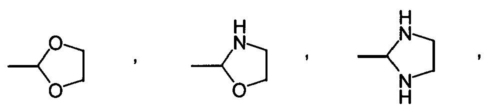 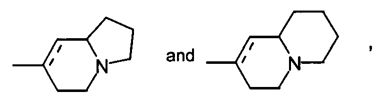 whereby each of these afore mentioned cyclic moieties may be substituted with 1, 2 or 3 substituent(s) independently selected from the group consisting of C₁₋₅-alkyl, -0-C₁₋₅-alkyl, -S-C₁₋₅-alkyl, oxo (=O), thia (=S), -C(=O)-O-C₁₋₅-alkyl, -O-C(=O)-C₁₋₅-alkyl, F, CI, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the NH groups; and if present, the dotted line represents an optional chemical bond;
or R³ represents a ―NR¹³R¹⁴ moiety;
more preferably R³ represents a ―NR¹³R¹⁴ moiety;
and n, R¹, R² and R⁴ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula I given above are preferred, wherein R⁴, R⁵, R⁶ and R⁷, identical or different, each represent a hydrogen atom; ―NO₂; -NH₂; -SH; -OH; -CN; -C(=O)-OH; -C(=O)-H; -S(=O)₂-OH; - C(=O)-NH₂; -S(=O)₂-NH₂; -C(=O)-R⁸ -S(=O)₂-R⁹; -OR¹⁰; -SR¹¹; -C(=O)-OR¹²; - N(R¹⁵)-S(=O)₂-R¹⁶; -NH-R¹⁷, -NR¹⁸R¹⁹; -C(=O)-NHR²⁰, -C(=O)-NR²¹R²²; -S(=O)₂₋NHR²³, -S(=O)₂-NR²⁴R²⁵; -O-C(=O)-R²⁶; -NH-C(=O)-R²⁷; -NR²⁸-C(=O)-R²⁹; NH-C(=O)-O-R³⁰; NR³¹-C(=O)-O-R³²; -S(=O)₂-O-R³³ ; F, Cl, Br, I; -CF₃, -CHF₂, -CH₂F, a linear or branched, unsubstituted C₁₋₁₀ alkyl radical; a linear or branched, unsubstituted C₂₋₆ alkenyl radical; a linear or branched, unsubstituted C₂₋₆ alkinyl radical; a saturated or unsaturated, optionally at least mono-substituted, 3- to 9-membered cycloaliphatic radical, which may be bonded via a linear or branched C₁₋₆₋alkylene group and/or which may contain 1, 2 or 3 heteroatom(s) independently selected from the group consisting of nitrogen, oxygen and sulphur as ring member(s); or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatom(s) independently selected from the group consisting of nitrogen, oxygen and sulphur as ring member(s);
preferably R⁴, R⁵, R⁶ and R⁷, identical or different, each represent a hydrogen atom; ―NO₂; -NH₂; -SH; -OH; -CN; -C(=O)-OH; -C(=O)-H; -S(=O)₂-OH; -C(=O)-NH₂; - S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, -NR¹⁸R¹⁹; -C(=O)-NHR²⁰, -C(=O)-NR²¹R²²; -S(=O)₂-NHR²³, -S(=O)₂₋NR²⁴R²⁵ ; -O-C(=O)-R²⁶; -NH-C(=O)-R²⁷; -NR²⁸-C(=O)-R²⁹; NH-C(=O)-O-R³⁰; NR³¹⁻C(=O)-O-R³²; -S(=O)₂-O-R³³; F, Cl, Br, l; -CF₃, -CHF₂, -CH₂F, an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅₋alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ― (CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
more preferably R⁴, R⁵, R⁶ and R⁷, identical or different, each represent a hydrogen atom; ―NO₂; -NH₂; -SH; -OH; -CN; -C(=O)-OH; -C(=O)-H; -S(=O)₂-OH; -C(=O)-NH₂; - S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, -NR¹⁸R¹⁹; F, Cl, Br, I; -CF₃, -CHF₂, -CH₂F, an unsubstituted alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a - (CH₂)_{1, 2 or 3}- group and/or may be substituted with 1, 2, 3, 4 or 5 substituent(s) independently selected from the group consisting of C₁₋₅-alkyl, -O-C₁₋₅-alkyl, -S-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;

in each case with the proviso that at least one of the substituents R⁴, R⁵, R⁶ and R⁷ represents an -N(R¹⁵)-S(=O)₂-R¹⁶ moiety;
and n, R¹ to R³ and R⁸ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Further preferred compounds of general formula I given above are such compounds, wherein one of the substituents R⁴, R⁵, R⁶ and R⁷ represents an -N(R¹⁵)-S(=O)₂-R¹⁶ moiety and at least one of the further substituents of R⁴, R⁵, R⁶ and R⁷ does not represent hydrogen; preferably one of the substituents R⁴, R⁵, R⁶ and R⁷ represents an -N(R¹⁵)-S(=O)₂-R¹⁶ moiety and one of the further substituents of R⁴, R⁵, R⁶ and R⁷ does not represent hydrogen; and n and the remaining substituents of R¹ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore such indole compounds of general formula I are preferred, wherein R⁴ represents an -N(R¹⁵)-S(=O)₂-R¹⁶ moiety and at least one of the substituents R⁵, R⁶ and R⁷ does not represent hydrogen; preferably R⁴ represents an -N(R¹⁵)-S(=O)₂-R¹⁶ moiety and one of the substituents R⁵, R⁶ and R⁷ does not represent hydrogen; and n and the remaining substituents of R¹ to R³ and R⁵ to R³³ have the above defined meaning optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Furthermore such indole compounds of general formula I are preferred, wherein R⁵ represents an -N(R¹⁵)-S(=O)₂-R¹⁶ moiety and at least one of the substituents R⁴, R⁶ and R⁷ does not represent hydrogen; preferably R⁵ represents an -N(R¹⁵)-S(=O)₂-R¹⁶ moiety and one of the substituents R⁴, R⁶ and R⁷ does not represent hydrogen; and n and the remaining substituents of R¹ to R⁴ and R⁶ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula I given above are preferred, wherein R⁶ represents an -N(R¹⁵)-S(=O)₂-R¹⁶ moiety and at least one of the substituents R⁴, R⁵ and R⁷ does not represent hydrogen; preferably R⁶ represents an -N(R¹⁵)-S(=O)₂-R¹⁶ moiety and one of the substituents R⁴, R⁵ and R⁷ does not represent hydrogen; and n and the remaining substituents of R¹ to R⁵ and R⁷ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula I given above are preferred, wherein R⁷ represents an -N(R¹⁵)-S(=O)₂-R¹⁶ moiety and at least one of the substituents R⁴, R⁵ and R⁶ does not represent hydrogen; preferably R⁷ represents an -N(R¹⁵)-S(=O)₂-R¹⁶ moiety and one of the substituents R⁴, R⁵ and R⁶ does not represent hydrogen; and n and the remainung substituents of R¹ to R⁶ and R⁸ to R³³ have the above defined meaning optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula I given above, wherein R⁸ represents a hydrogen atom or a linear or branched, optionally at least mono-substituted C₁₋₁₀ alkyl radical, preferably R⁸ represents a hydrogen atom or an alkyl radical selected from the group consisting of methyl, ethyl, n-propyl, iso-propyl, n-butyl, iso-butyl, sec-butyl and tert.-butyl, and n, R¹ to R⁷and R⁹ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula I given above, wherein R¹², R¹⁷, R¹⁸, R¹⁹, R²⁰, R²¹, R²², R²³, R²⁴, R²⁵, R²⁶, R²⁷, R²⁸, R²⁹, R³⁰, R³¹, R³² and R³³, independent from one another, each represent a linear or branched, unsubstituted C₁₋₁₀ alkyl radical; a linear or branched, unsubstituted C₂₋₆ alkenyl radical; a linear or branched, unsubstituted C₂₋₆alkinyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatom(s) independently selected from the group consisting of nitrogen, oxygen and sulphur as ring member(s); or an optionally at least mono-substituted 5- to 10-membered aryl or heteroaryl radical, which may be bonded via a linear or branched C₁₋₆-alkylene, C₂₋₆-alkenylene or C₂₋₆-alkinylene group and wherein the heteroaryl radical contains 1, 2 or 3 heteroatom(s) independently selected from the group consisting of nitrogen, oxygen and sulphur as ring member(s);
preferably R¹², R¹⁷, R¹⁸, R¹⁹, R²⁰, R²¹, R²², R²³, R²⁴, R²⁵, R²⁶, R²⁷, R²⁸, R²⁹, R³⁰, R³¹, R³² and R³³, independent from one another, each represent an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH₂)_{1, 2 or 3}- group and/or may be substituted with 1, 2, 3, 4 or 5 substituent(s) independently selected from the group consisting of C₁₋₅-alkyl, -O-C₁₋₅₋alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a moiety selected from the group consisting of ―(CH₂)-, -(CH₂)₂-, -(CH₂)₃- and ― (CH=CH)- and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
and n, R¹ to R¹¹ and R¹³ to R¹⁶ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula I given above, wherein R⁹ represents an optionally at least mono-substituted 5- to 10-membered aryl or heteroaryl radical, which may be bonded via a linear or branched C₁₋₆ alkylene, C₂₋₆ alkenylene or C₂₋₆ alkinylene group and wherein the heteroaryl radical contains 1, 2 or 3 heteroatom(s) independently selected from the group consisting of nitrogen, oxygen and sulphur as ring member(s) and which may be condensed with an optionally at least mono-substituted mono- or bicyclic ring system;
whereby the rings of the ring system are 5- 6- or 7-membered and may contain 1, 2 or 3 heteroatom(s) independently selected from the group consisting of nitrogen, oxygen and sulphur,
preferably R⁹ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ―(CH₂)_{1, 2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅₋alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
and n, R¹ to R⁸ and R¹⁰ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula I given above, wherein R¹⁰ and R¹¹, independent from one another, each represent a linear or branched, unsubstituted C₁₋₁₀ alkyl radical; a linear or branched, unsubstituted C₂₋₆ alkenyl radical; a linear or branched, unsubstituted C₂₋₆ alkinyl radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatom(s) independently selected from the group consisting of nitrogen, oxygen and sulphur as ring member(s);
preferably R¹⁰ and R¹¹, independent from one another, each represent an unsubstituted alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)₁,_{2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅-alkyl, - S(=O)₂-phenyl, cyclopropyl, cyclobutyl, cyclopentyl, cyclohexyl, phenyl, phenoxy and benzyl;
and n, R¹ to R⁹ and R¹² to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula I given above are preferred, wherein R¹³ and R¹⁴, independent from one another, each represent a hydrogen atom; a linear or branched, unsubstituted C₁₋₁₀ alkyl radical; a linear or branched, unsubstituted C₂₋₆ alkenyl radical; or a linear or branched, unsubstituted C₂₋₆ alkinyl radical;
or R¹³ and R¹⁴ together with the bridging nitrogen form an optionally at least mono-substituted, saturated or unsaturated, but not aromatic 3- to 9-membered heterocyclic ring which may contain 1, 2 or 3 heteroatom(s) independently selected from the group consisting of nitrogen, oxygen and sulphur as ring member(s) and/or which may be condensed with an optionally at least mono-substituted mono- or bicyclic ring system,
whereby the rings of the ring system are 5- 6- or 7-membered and may contain 1, 2 or 3 heteroatom(s) independently selected from the group consisting of nitrogen, oxygen and sulphur;
preferably R¹³ and R¹⁴, independent from one another, each represent a hydrogen atom; or an unsubstituted alkyl radical selected from the group consisting of methyl, ethyl, n-propyl, iso-propyl, n-butyl, sec-butyl, iso-butyl and tert-butyl;
R¹³ and R¹⁴ together with the bridging nitrogen atom form a moiety selected from the group consisting of 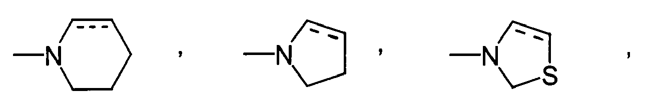 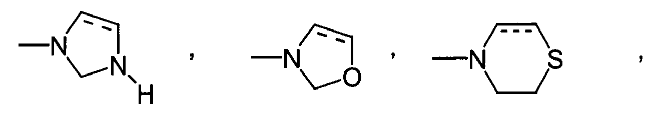 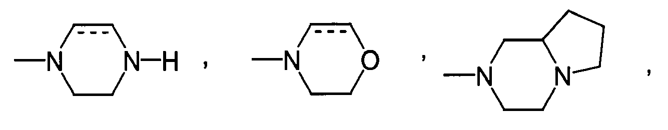 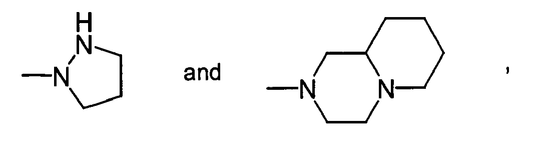
whereby each of these afore mentioned cyclic moieties may be substituted with 1, 2 or 3 substituent(s) independently selected from the group consisting of C₁₋₅-alkyl, -0-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the NH groups; and if present, the dotted line represents an optional chemical bond;
and n, R¹ to R¹² and R¹⁵ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Preferably the following proviso may apply to any definition of the substituents R¹³ and R¹⁴ given herein, namely that R¹³ and R¹⁴ do not both represent a hydrogen atom.

Also preferred are such substituted indole compounds of general formula I given above, wherein R¹⁵ represents a hydrogen atom; a linear or branched, unsubstituted C₁₋₁₀ alkyl radical; a linear or branched, unsubstituted C₂₋₆ alkenyl radical; a linear or branched, unsubstituted C₂₋₆ alkinyl radical or a ―S(=O)₂-R¹⁶ moiety;
preferably R¹⁵ represents a hydrogen atom; an unsubstituted alkyl radical selected from the group consisting of methyl, ethyl, n-propyl, iso-propyl, n-butyl, sec-butyl, iso-butyl and tert-butyl or a ―S(=O)₂-R¹⁶ moiety;
more preferably R¹⁵ represents a hydrogen atom;
and n, R¹ to R¹⁴ and R¹⁶ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula I given above, wherein R¹⁶ represents a linear or branched, unsubstituted C₁₋₁₀ alkyl radical; a linear or branched, unsubstituted C₂₋₆ alkenyl radical; a linear or branched, unsubstituted C₂₋₆ alkinyl radical; a saturated or unsaturated, optionally at least mono-substituted 3- to 9-membered cycloaliphatic radical, which may be bonded via a linear or branched C₁₋₆ alkylene, C₂₋₆ alkenylene or C₂₋₆ alkinylene group and/or which may contain 1, 2 or 3 heteroatom(s) independently selected from the group consisting of nitrogen, oxygen and sulphur as ring member(s); an optionally at least mono-substituted 5- to 10-membered aryl or heteroaryl radical, which may be bonded via a linear or branched C₁₋₆ alkylene, C₂₋₆ alkenylene or C₂₋₆ alkinylene group and wherein the heteroaryl radical contains 1, 2 or 3 heteroatom(s) independently selected from the group consisting of nitrogen, oxygen and sulphur as ring member(s) and which may be condensed with an optionally at least mono-substituted mono- or bicyclic ring system;
whereby the rings of the ring system are 5- 6- or 7-membered and may contain 1, 2 or 3 heteroatom(s) independently selected from the group consisting of nitrogen, oxygen and sulphur,
preferably R¹⁶ represents an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via moiety selected from the group consisting of ―(CH₂)-, -(CH₂)₂-, -(CH₂)₃- and ―(CH=CH)-and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -O-C(=O)-C₁₋₅-alkyl, F, CI, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ―(CH₂)₁, ₂ or ₃- group and/or may be substituted with 1, 2, 3, 4 or 5 substituent(s) independently selected from the group consisting of C₁₋₅-alkyl, -O-C₁₋₅-alkyl, -S-C₁₋₅₋alkyl, oxo (=O), thia (=S), -C(=O)-O-C₁₋₅-alkyl, -O-C(=O)-C₁₋₅-alkyl, F, CI, Br, I, -CN, - CF₃, -OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, cyclopropyl, cyclobutyl, cyclopentyl, cyclohexyl, phenyl, phenoxy and benzyl;
and n, R¹ to R¹⁵ and R¹⁷ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Moreover, such substituted indole compounds of general formula I are preferred, wherein n is 0, 1 or 2; preferably n is 0 or 1, more preferably n is 0; and R¹ to R³³ have the above defined meaning, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Also preferred are such substituted indole compounds of general formula I given above, wherein
n is 0,
R¹ represents a hydrogen atom,
R² represents a hydrogen atom,
R³ represents a ―NR¹³R¹⁴ moiety,
R⁴, R⁵, R⁶ and R⁷, identical or different, each represent a hydrogen atom; ―NO₂; - NH₂; -SH; -OH; -CN; -C(=O)-OH; -C(=O)-H; -S(=O)₂-OH; -C(=O)-NH₂; -S(=O)₂-NH₂; - C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, - NR¹⁸R¹⁹; F, CI, Br, I; -CF₃, -CHF₂, -CH₂F, an unsubstituted alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)₁,_{2 or 3-}group and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, l, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
with the proviso that one of the substituents R⁴, R⁵, R⁶ and R⁷ represents an -N(R¹⁵)-S(=O)₂-R¹⁶ moiety,
R⁸, R¹², R¹⁷ to R¹⁹, independent from one another, each represent an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH2)_{1,2 or 3}- group and/or may be substituted with 1, 2, 3, 4 or 5 substituent(s) independently selected from the group consisting of C₁₋₅-alkyl, -O-C₁₋₅₋alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a moiety selected from the group consisting of ―(CH₂)-, -(CH₂)₂-, -(CH₂)₃- and ― (CH=CH)- and/or may be substituted with 1, 2, 3, 4 or 5 substituent(s) independently selected from the group consisting of C₁₋₅-alkyl, -O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH_{2,} -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
R⁹ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅₋alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
R¹⁰ and R¹¹, independent from one another, each represent an unsubstituted alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ― (CH₂)_{1, 2 or 3}- group and/or may be substituted with 1, 2, 3, 4 or 5 substituent(s) independently selected from the group consisting of C₁₋₅-alkyl, -O-C₁₋₅-alkyl, -S-C₁₋₅₋alkyl, -C(=O)-O-C₁₋₅-alkyl, -O-C(=O)-C₁₋₅-alkyl, F, Cl, Br, l, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl,
R¹³ and R¹⁴, independent from one another, each represent a hydrogen atom; or an unsubstituted alkyl radical selected from the group consisting of methyl, ethyl, n-propyl, iso-propyl, n-butyl, sec-butyl, iso-butyl and tert-butyl,
or
R¹³ and R¹⁴ together with the bridging nitrogen atom form a moiety selected from the group consisting of 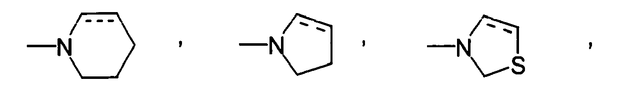 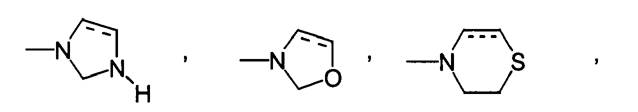 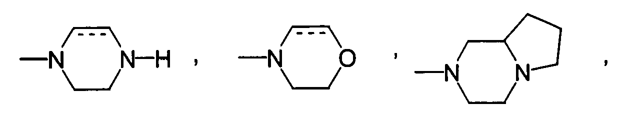 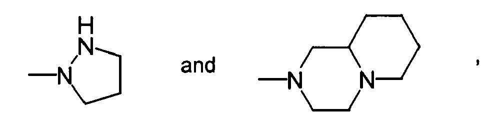
whereby each of these afore mentioned cyclic moieties may be substituted with 1, 2 or 3 substituent(s) independently selected from the group consisting of C₁₋₅-alkyl, -0-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)2-C₁₋₅-alkyl, -S(=O)₂-phenyl, cyclopropyl, cyclobutyl, cyclopentyl, cyclohexyl, phenyl, phenoxy and benzyl in any position including the NH groups; and, if present, the dotted line represents an optional chemical bond,
R¹⁵ represents a hydrogen atom
and
R¹⁶ represents an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via moiety selected from the group consisting of ―(CH₂)-, -(CH₂)₂-, -(CH₂)₃- and ―(CH=CH)- and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -0-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

In yet another aspect the present invention relates to substituted indole compounds of general formula I' 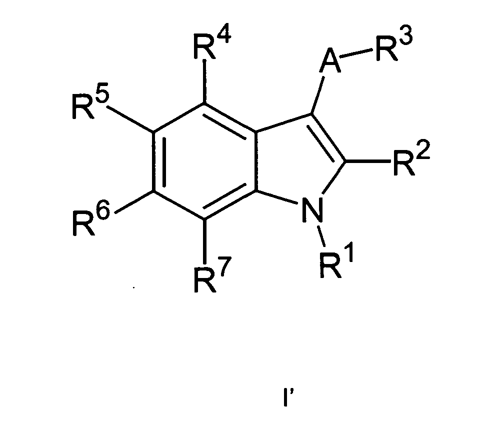
wherein A represents an alkylene group with 1, 2, 3 or 4 carbon atoms in the chain, wherein the chain may be at least mono-substituted and the remaining substituents n and R¹-R⁷ are as defined above, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferably the alkylene group may be substituted by one or more substituents, preferably by 1, 2 or 3, more preferably by one substituent independently selected from the group consisting of -O-C₁₋₅-alkyl, -S-C₁₋₅-alkyl, -F, Cl, Br, I, -CN, -CF₃, - OCF₃, -SCF₃, -OH, -SH, -NH₂, -NH(C₁₋₅-alkyl) and -N(C₁₋₅-alkyl)₂ , whereby in each occurence C₁₋₅-alkyl may be linear or branched.

Particularly preferred are substituted indole compounds of general formula I or I' selected from the group consisting of

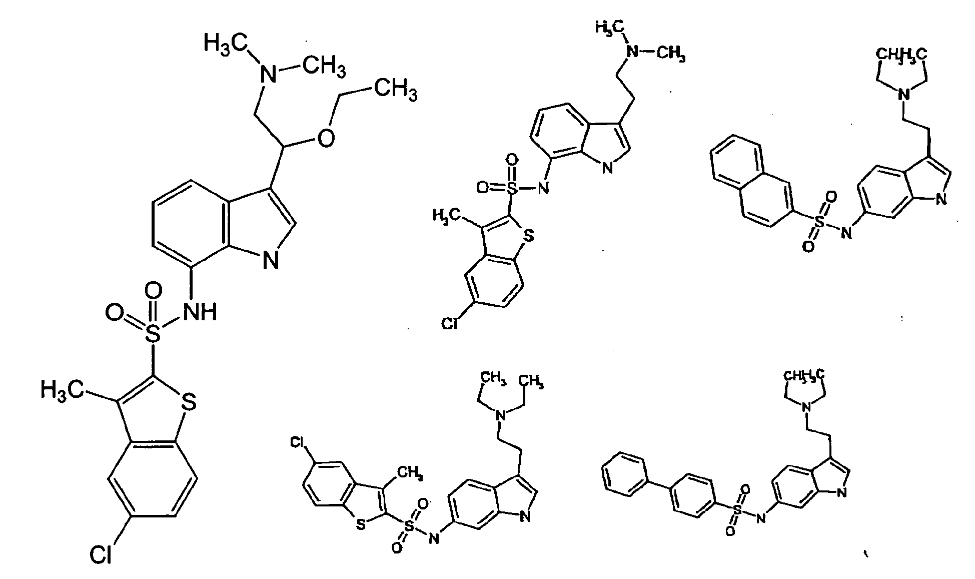

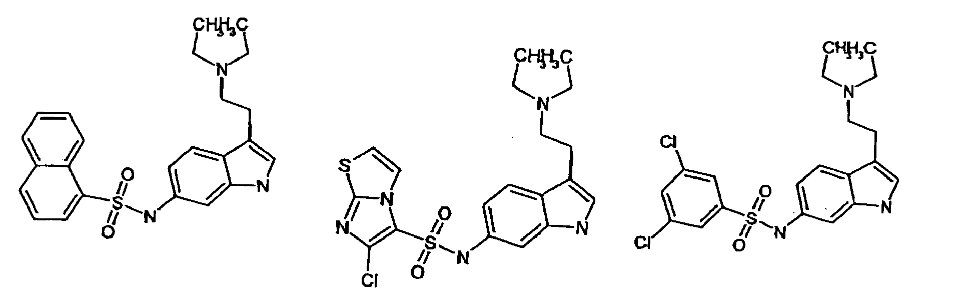

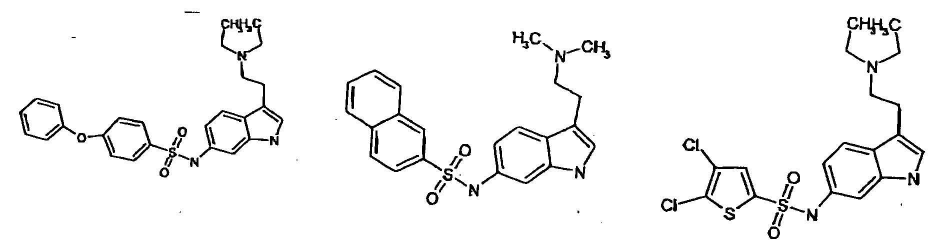

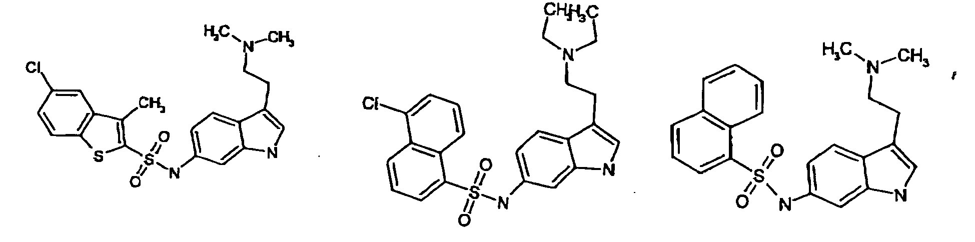

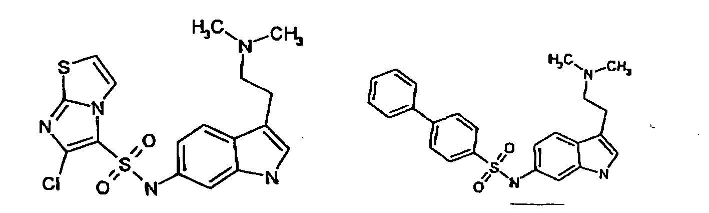

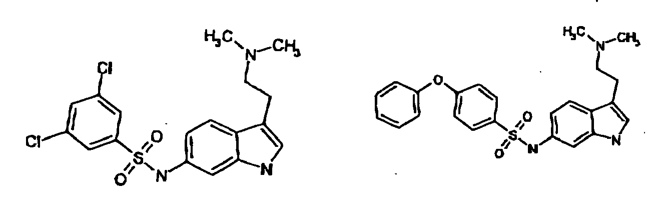

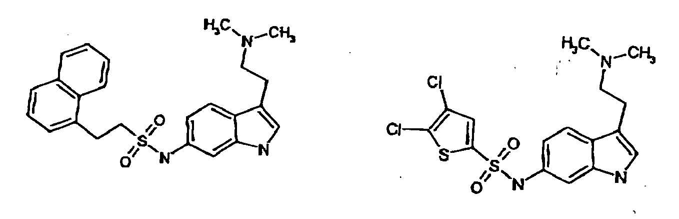

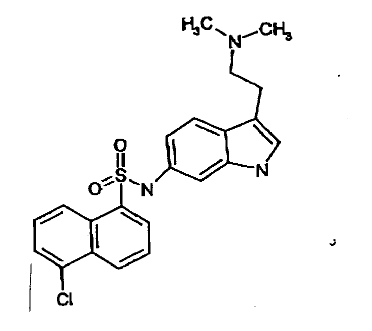

Another aspect of the present invention relates to a process for the preparation of a substituted indole compound of general formula I, wherein at least one compound of general formula II, 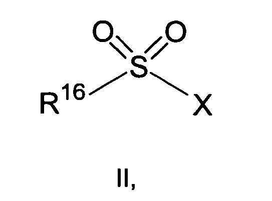
wherein R¹⁶ has the meaning given above and X represents a leaving group, preferably a halogen atom, particularly preferably a chlorine atom, is reacted with at least one compound of general formula III, 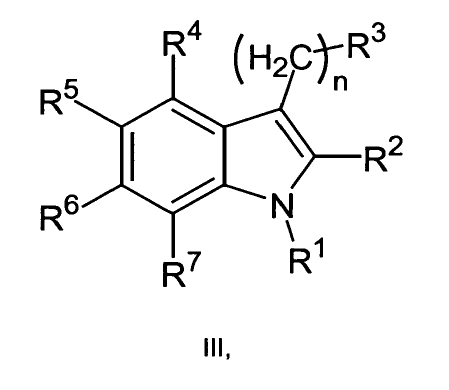
wherein R¹ to R⁷ and n have the meaning given above, with the proviso that at least one substituent of the group consisting of R⁴, R⁵, R⁶ and R⁷ represents a ―N(H)(R¹⁵) moiety or a protected derivative thereof, in a suitable reaction medium, preferably in the presence of at least one base.

If a protected derivative is used, the respective protective group has to be removed after the reaction to obtain the desired substituted indole compound of general formula I. Suitable protecting groups as well as methods for introducing and removing such protecting groups are well known to those skilled in the art as described below.

Suitable reaction media for the reaction between compounds of general formulas II and III include organic solvents, such as dialkyl ether, preferably diethyl ether, or a cyclic ether, preferably tetrahydrofuran or dioxane; or a halogenated hydrocarbon, preferably dichloromethane or chloroform; an alcohol, preferably methanol or ethanol; a dipolar aprotic solvent, preferably acetonitrile, pyridine or dimethylformamide, or any other suitable reaction medium. Of course, mixtures of at least two classes of solvents or of at least two solvents of one class may also be used.

The reaction is preferably carried out in the presence of at least one suitable base, for example, an inorganic base such as a hydroxide or a carbonate of an alkali metal and/or an organic base, preferably triethylamine or pyridine.

The reaction is preferably carried out at a temperature between ― 10°C and ambient temperature, i.e. approximately 25 °C and the reaction time is preferably between 5 minutes and 24 hours.

The compounds of general formula I given above may be purified and/or isolated according to methods well known to those skilled in the art. Preferably, the compounds of general formula I may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

The compounds of general formula II are commercially available or may be prepared according to methods well known in the art, for example, analogous to the methods described in the bibliography of E.E. Gilbert, Synthesis, 1969, 1, 3. The respective part of the literature description cited above is hereby incorporated by reference and forms part of the disclosure.

The compounds of general formula III are commercially available or may also be prepared according to standard methods known in the prior art, for example by methods similar to those described in the literature: Pigerol, Charles; De Cointet de Fillain, Paul; Eymard, Pierre; Werbenec, Jean Pierre; Broll, Madeleine. (Labaz S. A., France), DE 2727047; Schwink, Lothar; Stengelin, Siegfried; Gossel, Matthias. "Preparation of indol-5-ylureas and related compounds for the treatment of obesity and type II diabetes", WO 0315769 A1. One of them consists of nitro group reduction of derivatives of general formula IV, 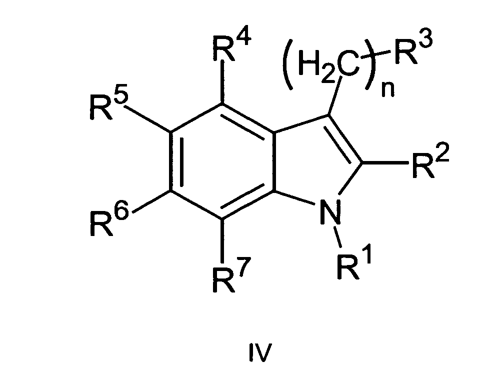
wherein R¹ to R⁷ and n have the meaning given above; with the proviso that at least one substituent of the group consisting of R⁴, R⁵, R⁶ and R⁷ represents ―NO₂; or one of their suitably protected derivatives by methods known in the prior art, as for example: BRATTON, L. D.; ROTH, B. D.; TRIVEDI, B. K.; UNANGST, P. C.; J Heterocycl Chem , 2000, 37 (5), 1103-1108. FANGHAENEL, E.; CHTCHEGLOV, D.; J Prakt Chem/Chem-Ztg, 1996, 338 (8), 731-737. KUYPER, L. F.; BACMAYARI, D. P.; JONES, M. L.; HUNTER, R. N.; TANSIK, R. L.; JOYNER, S. S.; BOYTOS, C. M.; RUDOLPH, S. K.; KNICK, V.; WILSON, H. R.; CADDELL, J. M.; FRIEDMAN, H. S.; ET AL.; J Med Chem, 1996, 39 (4), 892-903; and, if necessary, the protective groups are removed in order to obtain the corresponding amine of general formula lll, which may be purified and/or isolated by means of conventional methods known in the prior art. The respective parts of the literature descriptions cited above are hereby incorporated by reference and form part of the disclosure.

The compounds of general formula IV are commercially available or may also be prepared according to standard methods known in the prior art, for example by methods similar to those described in the literature: Journal of Heterocyclic Chemistry, 37(5), 1103-1108; 2000; Schwink, Lothar; Stengelin, Siegfried; Gossel, Matthias, "Preparation of indol-5-ylureas and related compounds for the treatment of obesity and type II diabetes", WO 0315769 A1, Baxter, Andrew; Brough, Stephen; Mcinally, Thomas; Mortimore, Michael; Cladingboel, David, "Preparation of N-aryl-1-adamantaneacetamides and analogs as purinergic P2Z receptor antagonists" WO 9929660 A1; Pigerol, Charles; De Cointet de Fillain, Paul; Eymard, Pierre; Werbenec, Jean Pierre; Broll, Madeleine, "Indole derivatives", DE 2727047. The respective parts of the literature descriptions cited above are hereby incorporated by reference and form part of the disclosure.

Another method consists of the alkylation of nitro derivatives of general formula V, 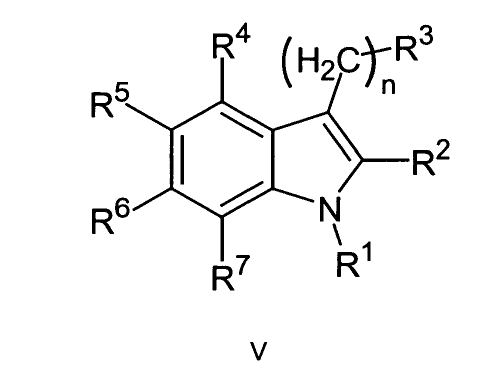
wherein R² to R⁷and n have the meaning given above and R¹ represents a hydrogen atom; with the proviso that at least one substituent of the group consisting of R⁴, R⁵, R⁶ and R⁷ represents ―NO₂; or one of their suitably protected derivatives by methods known in the prior art, as for example: BHAGWAT, S. S.; GUDE, C.; Tetrahedron Lett, 1994, 35 (12), 1847-1850. BRATTON, L. D.; ROTH, B. D.; TRIVEDI, B. K.; UNANGST, P. C.; J Heterocycl Chem, 2000, 37 (5), 1103-1108; and, if necessary, the protective groups are removed in order to obtain the corresponding amine of general formula III, which may be purified and/or isolated by means of conventional methods known in the prior art. The respective parts of the literature descriptions cited above are hereby incorporated by reference and form part of the disclosure.

The compounds of general formula V are commercially available or may also be prepared according to standard methods known in the prior art, as for example YAMASHKIN, S. A.; YUROVSKAYA, M. A.; Chem Heterocycl Compd (N Y) 1999, 35 (12), 1426-1432. OTTONI, O.; CRUZ, R.; KRAMMER, N. H.; Tetrahedron Lett ,1999, 40 (6), 1117-1120. EZQUERRA, J.; PEDREGAL, C.; LAMAS, C.; BARLUENGA, J.; PEREZ, M.; GARCIA-MARTIN, M. A.; GONZALEZ, J. M.; J Org Chem ,1996, 61 (17), 5804-5812. FADDA, A. A.; Indian J Chem, Sect B: Org Chem Incl Med Chem , 1990, 29 (11),1017-1019. KATRITZKY, A. R.; RACHWAL, S.; BAYYUK, S.; Org Prep Proced lnt, 1991, 23 (3), 357-363. Inada, A.; Nakamura, Y.; Morita, Y.; Chem Lett , 1980, 1287.

The respective literature descriptions are incorporated by reference and form part of the disclosure.

Alternatively, the substituted indole compounds of general formula I given above, in which R¹⁵ represents a linear or branched, saturated or unsaturated, optionally at least mono-substituted aliphatic radical and all of the other substituents have the above defined meaning, may also be prepared by an alkylation type reaction from the corresponding compound of general formula I; wherein R¹ to R⁷ and n have the meaning as given above with the proviso that at least one substituent of the group consisting of R⁴, R⁵, R⁶ and R⁷ represents ―NH-S(=O)-R¹⁶; e.g. by reaction with corresponding halide R¹⁵-X, wherein X represents a halogen atom, preferably a chlorine atom, or a sulfate of the general formula VI, 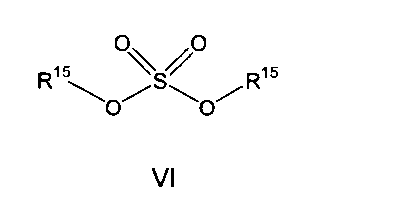
wherein in each case R¹⁵ has the afore mentioned meaning. The corresponding halides and sulfates are commercially available or may be prepared according to methods well known to those skilled in the art.

The alkylation type reaction is preferably carried out in the presence of at least one suitable base such as a hydroxide and/or a carbonate of an alkali metal, a metal hydride, alcoxides such as sodium methoxide or potassium tert-butoxid, organometallic compounds such as n-butyllithium or tert-butyllithium, in the presence of a suitable reaction medium such as dialkyl ether, preferably diethyl ether, or a cyclic ether, preferably tetrahydrofuran or dioxane, a hydrocarbon, preferably toluene, an alcohol, preferably methanol or ethanol, a dipolar aprotic solvent, preferably acetonitrile, pyridine or dimethylformamide, or any other suitable reaction medium. Of course, mixtures of at least two classes of solvents or of at least two solvents of one class may also be used.

The reaction is preferably carried out at a temperature between ―10 °C and ambient temperature, i.e. approximately 25 °C and the reaction time is preferably 1 and 24 hours.

The compounds of general formula I given above may be purified and/or isolated according to methods well known to those skilled in the art. Preferably, the compounds of general formula I may be isolated by evaporating the reaction medium, addition of water and then adjusting the pH value to obtain the compound in form of a solid that can be isolated by filtration, or by extraction with a solvent that is not miscible with water such as chloroform and purified by chromatography or recrystallisation from a suitable solvent.

During some synthetic reactions described above or while preparing the compounds of general formulas I, II , lll, IV, V or Vl the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted indole compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents.

The substituted indole derivatives of general formula I and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above. Suitable inorganic acids include but are not limited to hydrochloric acid, hydrobromic acid, phosphoric acid, sulfuric acid, nitric acid, suitable organic acids include but are not limited to citric acid, maleic acid, fumaric acid, tartaric acid, or derivatives thereof, p-toluenesulfonic acid, methanesulfonic acid or camphersulfonic acid.

Solvates, preferably hydrates, of the substituted indole compounds of general formula I or in each case of corresponding stereoisomers may also be obtained by standard procedures known to those skilled in the art.

A further aspect of the present invention relates to a medicament comprising at least one substituted indole compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one auxiliary agent.

Said medicament is particularly suitable for 5-HT₆-receptor regulation and therefore for the prophylaxis and/or treatment of a disorder or a disease that is least partially mediated via 5-HT₆-receptors.

Preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; or hyperactivity disorder (ADHD, attention deficit/hyperactivity disorder) or for the improvement of cognition (cognitive enhancement).

More preferably said medicament is suitable for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Furthermore, more preferably said medicament is suitable for the improvement of cognition (for cognitive enhancement).

Most preferably, said medicament is suitable for the prophylaxis and/or treatment of obesity and/or disorders or diseases related thereto.

In another aspect the present invention relates to the use of at least one substituted indole compound of general formula I given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for 5-HT₆-receptor regulation, preferably for the prophylaxis and/or treatment of a disorder or a disease that is least partially mediated via 5-HT₆-receptors.

The use of at least one substituted indole compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity, or for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; or hyperactivity disorder (ADHD, attention deficit/hyperactivity disorder) or for improvement of cognition (cognitive enhancement) is preferred.

More preferred is the use of at least one substituted indole compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or a disease that is related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

Most preferred is the use of at least one substituted indole compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for for the prophylaxis and/or treatment of obesity and/or disorders or diseases related thereto.

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are are known from the prior art, e.g. from the table of contents of"Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective indole compound is liberated in the intestinal tract. Preferably the enteric coating is soluble at a pH value of 5 to 7.5. Suitable materials and methods for the preparation are are known from the prior art.

Typically, the medicaments according to the present invention may contain 1-60 % by weight of one or more substituted indole compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

In the following methods for determining the pharmacological activity of the substituted indole compounds are described.

### PHARMACOLOGICAL METHODS:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted indole compounds according to the present invention in fasted rats is then determined as follows:

The rats were fasted for 23 hours in their single homecages. After this period, the rats are orally or intraperitoneally dosed with a composition comprising a substituted indole compound or a corresponding composition (vehicle) without said substituted indole compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Tumbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Examples:

The compounds according to the following examples have been prepared as described above: 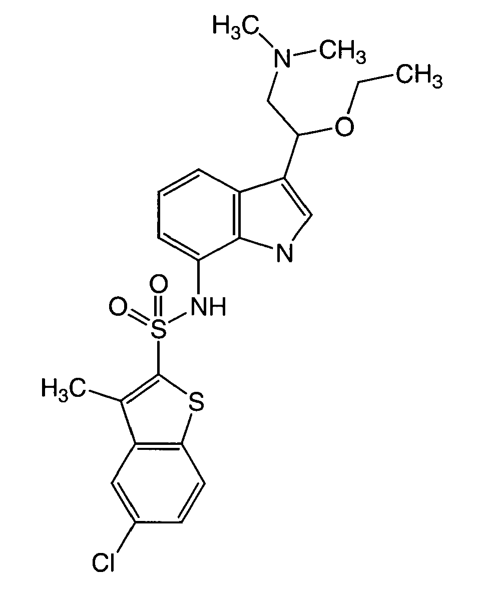
**NMR:**
1,02 (t, 3H, J=7,0 Hz); 2,33 (s, 3H); 2,42 (s, 6H); 2,79 (m, 1 H); 3,01 (m, 1 H); 4,76 (m, 1 H); 6,73 (m, 2H); 7,20 (d, 1 H, 2,5Hz); 7,30 (d, 1 H, J=7,2 Hz); 7,48 (dd, 1 H, J=8,8Hz, J`=1,9 Hz), 7,87 (d, 1 H, J=1,BHz); 7,99 (d, 1 H, J=8,6Hz); 10,70 (s, 1 H) in DMSO-d6
**Melting point: 145°C**

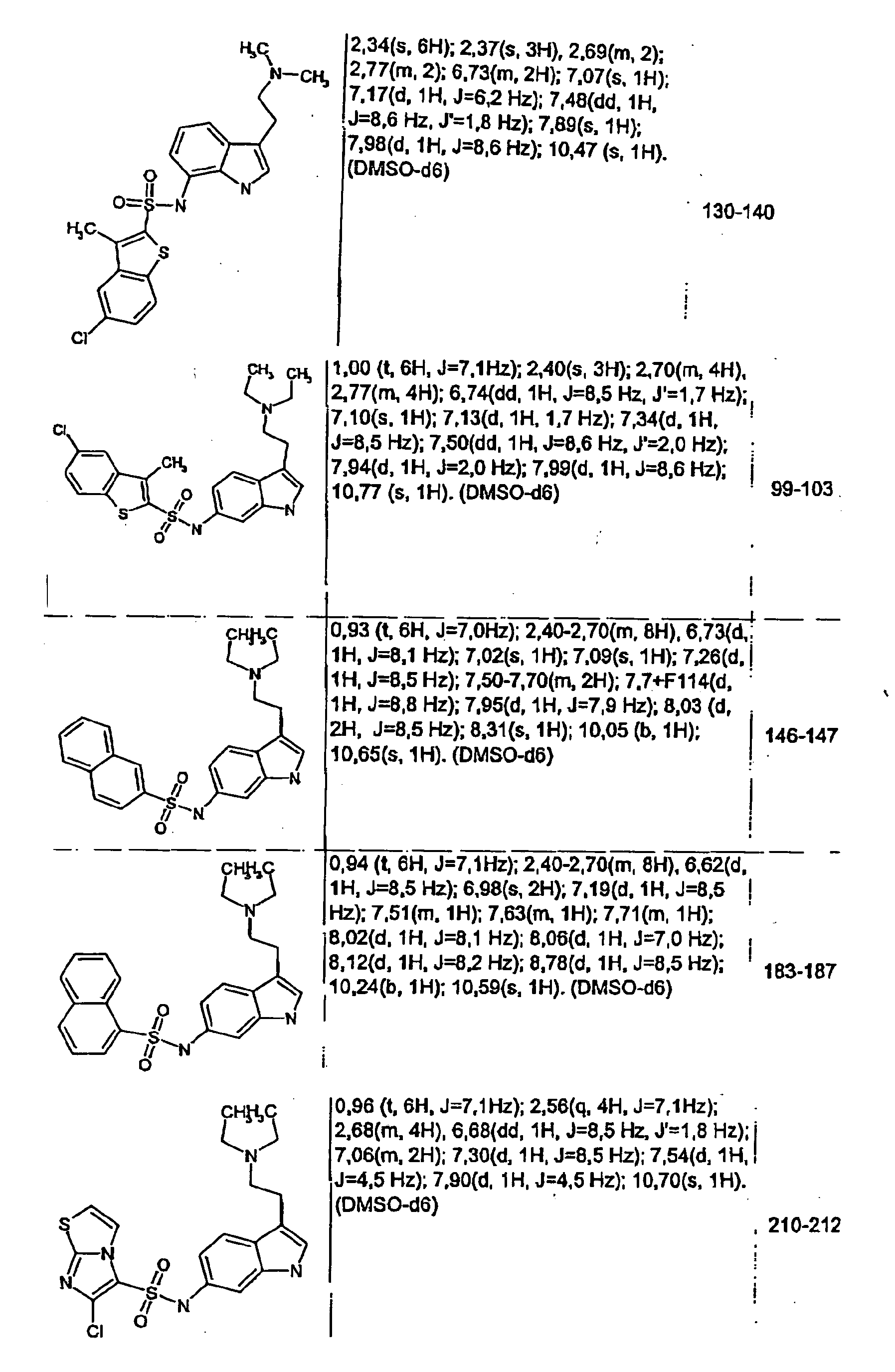

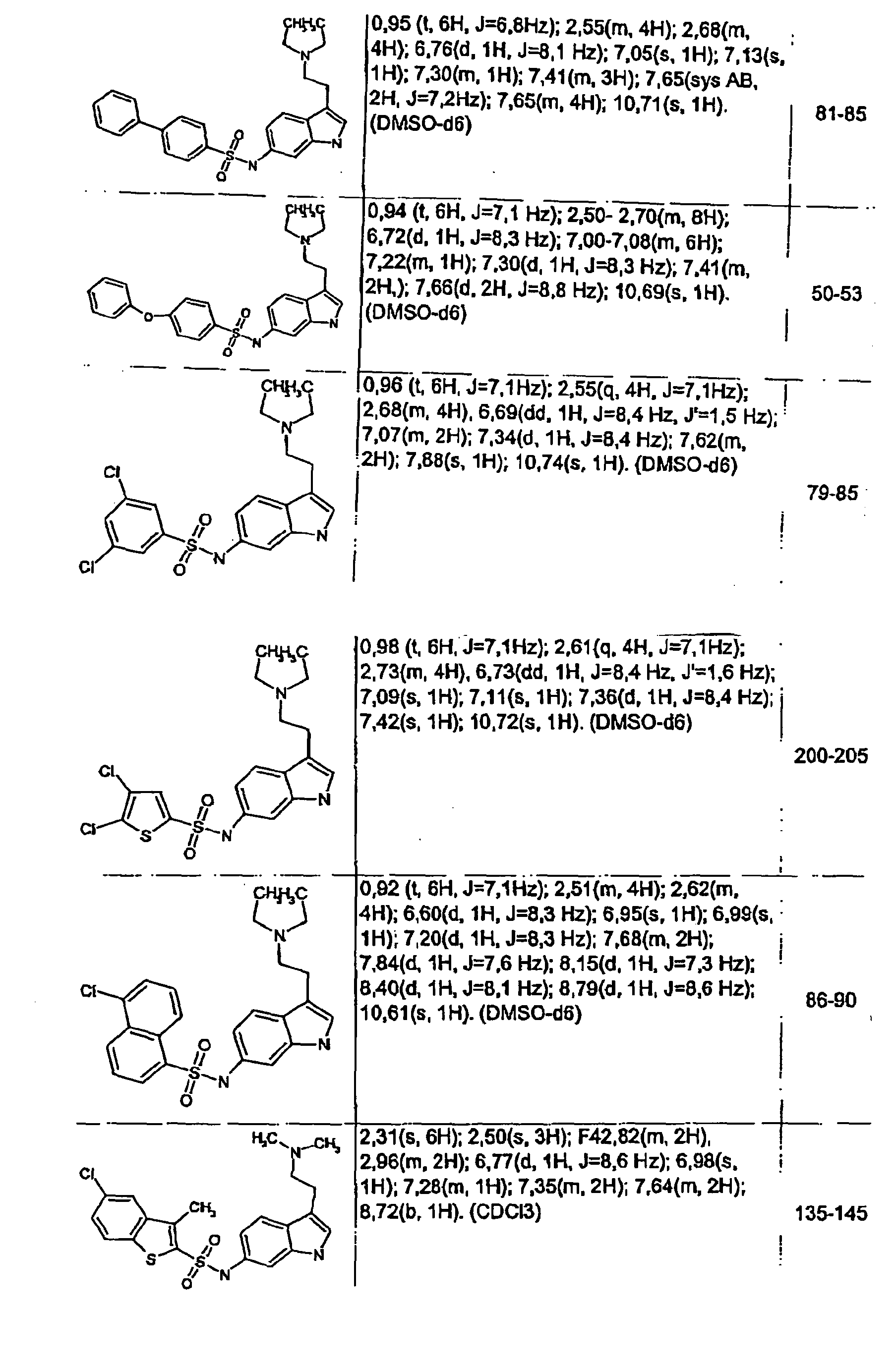

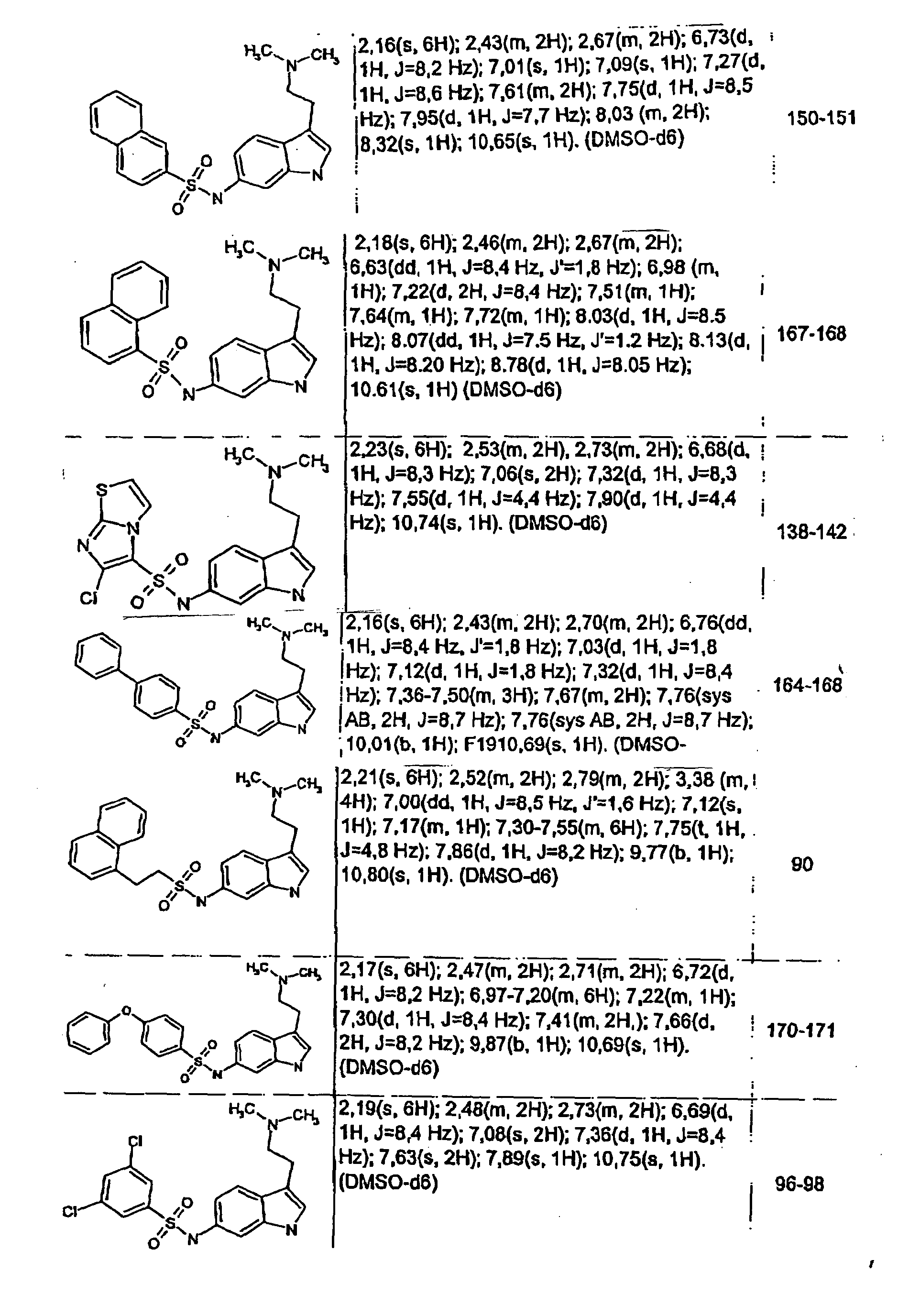

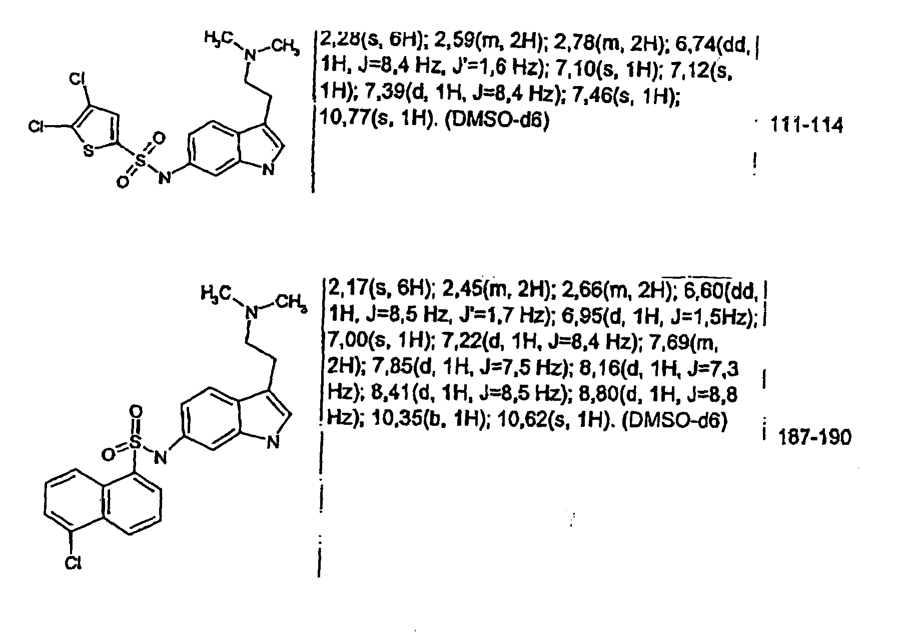

whereby the data given in the second column is the NMR data and the data given in the third column is the melting point.

## Claims

**1.** A substituted indole compound of general formula I, 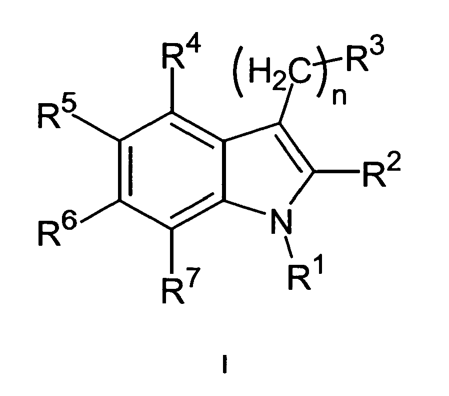
wherein
n is 0, 1, 2, 3 or 4,
R¹ represents a hydrogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical,
which may be bonded via a linear or branched alkylene group; an optionally at least mono-substituted aryl or heteroaryl radical, which may be bonded via a linear or branched alkylene group; a ―C(=O)-R⁸ moiety; or a ―S(=O)₂-R⁹ moiety,
R² represents a hydrogen atom; ―NO₂; -NH₂; -SH; -OH; -CN; -C(=O)-OH; -0-R¹⁰; -S-R¹¹; -C(=O)-OR¹²; a halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
R³ represents a saturated or unsaturated, optionally at least mono-substituted, optionally at least one heteroatom as a ring member containing cycloaliphatic radical, which may be condensed with an optionally at least mono-substituted mono- or polycyclic ring system or a ―NR¹³R¹⁴ moiety,
R⁴, R⁵, R⁶ and R⁷, identical or different, each represent a hydrogen atom; ― NO₂; -NH₂; -SH; -OH; -CN; -C(=O)-OH; -C(=O)-H; -S(=O)₂-OH; -C(=O)-NH₂; - S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷; -NR¹⁸R¹⁹; -C(=O)-NHR²⁰, -C(=O)-NR²¹R²²; -S(=O)₂₋NHR²³, -S(=O)₂-NR²⁴R²⁵; -O-C(=O)-R²⁶; -NH-C(=O)-R²⁷; -NR²⁸-C(=O)-R²⁹; NH-C(=O)-O-R³⁰; NR³¹-C(=O)-O-R³²; -S(=O)₂-O-R³³; a halogen atom; a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene group,
with the proviso that at least one of the substituents R⁴, R⁵, R⁶ and R⁷ represents an -N(R¹⁵)-S(=O)₂-R¹⁶ moiety and at least one of the other substituents of R⁴, R⁵, R⁶ and R⁷ does not represent hydrogen,
R⁸ represents a hydrogen atom or a linear or branched, saturated or unsaturated, optionally at least mono-substituted aliphatic radical,
R¹², R¹⁷, R¹⁸, R¹⁹, R²⁰, R²¹, R²², R²³, R²⁴, R²⁵, R²⁶, R²⁷, R²⁸, R²⁹, R³⁰, R³¹, R³² and R³³, independent from one another, each represent a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched alkylene group; or an optionally at least mono-substituted aryl or heteroaryl radical, which may be bonded via a linear or branched alkylene, alkenylene or alkinylene group,
R⁹ represents an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system,
R¹⁰ and R¹¹, independent from one another, each represent a linear or branched, saturated or unsaturated, optionally at least mono-substituted aliphatic radical; or an optionally at least mono-substituted aryl or heteroaryl radical, which may be bonded via a linear or branched alkylene group,
R¹³ and R¹⁴, independent from one another, each represent a hydrogen atom; or a linear or branched, saturated or unsaturated, optionally at least mono-substituted aliphatic radical;
or
R¹³ and R¹⁴ together with the bridging nitrogen form an optionally at least mono-substituted, saturated, unsaturated or aromatic heterocyclic ring which may contain at least one further heteroatom as a ring member and/or which may be condensed with an optionally at least mono-substituted mono- or polycyclic ring system,
R¹⁵ represents a hydrogen atom; a linear or branched, saturated or unsaturated, optionally at least mono-substituted aliphatic radical or a ― S(=O)₂-R¹⁶ moiety,
and
R¹⁶ represents a linear or branched, saturated or unsaturated, optionally at least mono-substituted aliphatic radical; a saturated or unsaturated, optionally at least mono-substituted, optionally at least one heteroatom as a ring member containing cycloaliphatic radical, which may be bonded via a linear or branched, optionally at least mono-substituted alkylene, alkenylene or alkinylene group and/or which may be condensed with an optionally at least mono-substituted mono- or polycyclic ring system; or an optionally at least mono-substituted aryl or heteroaryl radical, which may be bonded via a linear or branched, optionally at least mono-substituted alkylene, alkenylene or alkinylene group and/or which may be condensed with an optionally at least mono-substituted mono- or polycyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

**2.** A compound according to claim 1, **characterised in that**
n is 0, 1, 2, 3 or 4,
R¹ represents a hydrogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; an optionally at least mono-substituted 5- to 14-membered aryl or heteroaryl radical, which may be bonded via a linear or branched C₁₋₆ alkylene group; a ―C(=O)-R⁸ moiety; or a ―S(=O)₂-R⁹ moiety,
R² represents a hydrogen atom; ―NO₂; -NH₂; -SH; -OH; -CN; -C(=O)-OH; -0-R¹⁰; -S-R¹¹; -C(=O)-OR¹² ; a halogen atom; a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
R³ represents a saturated or unsaturated, optionally at least mono-substituted, optionally at least one heteroatom as a ring member containing 3- to 9-membered cycloaliphatic radical, which may be condensed with an optionally at least mono-substituted mono- or polycyclic ring system or a -NR¹³R¹⁴ moiety,
R⁴, R⁵, R⁶ and R⁷, identical or different, each represent a hydrogen atom; ― NO₂; -NH₂; -SH; -OH; -CN; -C(=O)-OH; -C(=O)-H; -S(=O)₂-OH; -C(=O)-NH₂; - S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, -NR¹⁸R¹⁹; -C(=O)-NHR²⁰, -C(=O)-NR²¹R²²; -S(=O)₂₋NHR²³, -S(=O)₂-NR²⁴R²⁵; -O-C(=O)-R²⁶; -NH-C(=O)-R²⁷; -NR²⁸-C(-O)-R²⁹; NH-C(=O)-O-R³⁰; NR³¹-C(=O)-O-R³²; -S(=O)₂-O-R³³; a halogen atom; a linear or branched, saturated or unsaturated, optionally at least mono-substituted C₁₋10 aliphatic radical; a saturated or unsaturated, optionally at least mono-substituted, optionally at least one heteroatom as a ring member containing 3-to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene group,
with the proviso that at least one of the substituents R⁴, R⁵, R⁶ and R⁷ represents an -N(R¹⁵)-S(=O)₂-R¹⁶ moiety and at least one of the other substituents of R⁴, R⁵, R⁶ and R⁷ does not represent a hydrogen atom,
R⁸ represents a hydrogen atom or a linear or branched, saturated or unsaturated, optionally at least mono-substituted C₁₋₁₀ aliphatic radical,
R¹², R¹⁷ R¹⁸, R¹⁹, R²⁰, R²¹, R²², R²³, R²⁴, R²⁵, R²⁶, R²⁷, R²⁸, R²⁹, R³⁰, R³¹, R³² and R³³, independent from one another, each represent a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆ alkylene group; or an optionally at least mono-substituted 5- to 14-membered aryl or heteroaryl radical, which may be bonded via a linear or branched C₁₋₆ alkylene, C₂₋₆ alkenylene or C₂₋₆ alkinylene group,
R⁹ represents an optionally at least mono-substituted 5- to 14-membered aryl or heteroaryl radical, which may be bonded via a linear or branched optionally at least mono-substituted C₁₋₆ alkylene, C₂₋₆ alkenylene or C₂₋₆ alkinylene group and/or which may be condensed with an optionally at least mono-substituted mono- or polycyclic ring system,
R¹⁰ and R¹¹, independent from one another, each represent a linear or branched, saturated or unsaturated, optionally at least mono-substituted C₁₋₁₀ aliphatic radical; or an optionally at least mono-substituted 5- to 14-membered aryl or heteroaryl radical, which may be bonded via a linear or branched C₁₋₆ alkylene group,
R¹³ and R¹⁴, independent from one another, each represent a hydrogen atom; or a linear or branched, saturated or unsaturated, optionally at least mono-substituted C₁₋₁₀ aliphatic radical;
or
R¹³ and R¹⁴ together with the bridging nitrogen form an optionally at least mono-substituted, saturated, unsaturated or aromatic 3- to 9-membered heterocyclic ring which may contain at least one further heteroatom as a ring member and/or which may be condensed with an optionally at least mono-substituted mono- or polycyclic ring system,
R¹⁵ represents a hydrogen atom; a linear or branched, saturated or unsaturated, optionally at least mono-substituted C₁₋₁₀ aliphatic radical or a ― S(=O)₂-R¹⁶ moiety,
and
R¹⁶ represents a linear or branched, saturated or unsaturated, optionally at least mono-substituted C₁₋₁₀ aliphatic radical; a saturated or unsaturated, optionally at least mono-substituted, optionally at least one heteroatom as a ring member containing 3- to 9-membered cycloaliphatic radical, which may be bonded via a linear or branched C₁₋₆alkylene, C₂₋₆alkenylene or C₂₋₆ alkinylene group and/or which may be condensed with an optionally at least mono-substituted mono- or polycyclic ring system; or an optionally at least mono-substituted 5- to 14-membered aryl or heteroaryl radical, which may be bonded via a linear or branched C₁₋₆ alkylene, C₂₋₆ alkenylene or C₂₋₆ alkinylene group and/or which may be condensed with an optionally at least mono-substituted mono- or polycyclic ring system;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

**3.** A compound according to claim 1 or 2, **characterised in that** R¹ represents a hydrogen atom; a linear or branched, optionally at least mono-substituted C₁₋₁₀ alkyl radical; a linear or branched, optionally at least mono-substituted C₂₋₆ alkenyl radical; a linear or branched, optionally at least mono-substituted C₂₋₆ alkinyl radical; a saturated or unsaturated, optionally at least mono-substituted 3- to 9-membered cycloaliphatic radical, which may be bonded via a linear or branched C₁₋₆ alkylene group and/or which may contain 1, 2 or 3 heteroatom(s) independently selected from the group consisting of nitrogen, oxygen and sulphur as ring member(s); an optionally at least mono-substituted 5- to 10-membered aryl or heteroaryl radical, which may be bonded via a linear or branched C₁₋₆ alkylene group and wherein the heteroaryl radical contains 1, 2 or 3 heteroatom(s) independently selected from the group consisting of nitrogen, oxygen and sulphur as ring member(s); a ―C(=O)-R⁸ moiety; or a ―S(=O)₂-R⁹ moiety;
preferably R¹ represents a hydrogen atom; an unsubstituted alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -0-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)_{1, 2 or 3}- group and/or may be substituted with 1, 2, 3, 4 or 5 substituent(s) independently selected from the group consisting of C₁₋₅-alkyl, - O-C₁₋₅-alkyl, -S-C₁₋₅-alkyl, -C(=O)-O-C₁₋₅-alkyl, -O-C(=O)-C₁₋₅-alkyl, F, Cl, Br, l, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; a ―C(=O)-R⁸ moiety; or a ―S(=O)₂-R⁹ moiety;
more preferably R¹ represents a hydrogen atom.

**4.** A compound according to one or more of claims 1 to 3, **characterised in that** R² represents a hydrogen atom; ―NO₂; -NH₂; -SH; -OH; -CN; -C(=O)-OH; - CF₃; ―O-R¹⁰; -S-R¹¹; -C(=O)-OR¹²; F; Cl; Br; l; a linear or branched, unsubstituted C₁₋₁₀ alkyl radical; a linear or branched, unsubstituted C₂₋₆ alkenyl radical; a linear or branched, unsubstituted C₂₋₆ alkinyl radical; a saturated or unsaturated, optionally at least mono-substituted 3- to 9-membered cycloaliphatic radical, which may be bonded via a linear or branched C₁₋₆ alkylene group and/or which may contain 1, 2 or 3 heteroatom(s) independently selected from the group consisting of nitrogen, oxygen and sulphur as ring member(s); or an optionally at least mono-substituted aryl or heteroaryl radical, which may be bonded via a linear or branched C₁₋₆ alkylene group and wherein the heteroaryl radical contains 1, 2 or 3 heteroatom(s) independently selected from the group consisting of nitrogen, oxygen and sulphur as ring member(s);
preferably R² represents a hydrogen atom; ―NO₂; -NH₂; -SH; -OH; -CN; - C(=O)-OH; -CF₃; -O-R¹⁰; -S-R¹¹; -C(=O)-OR¹²; F; Cl; Br; I; an unsubstituted alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH₂)₁,_{2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -0-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ― (CH₂)_{1,2 or 3}- group and/or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-O-C₁₋₅-alkyl, -O-C(=O)-C₁₋₅-alkyl, F, Cl, Br, l, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl,
more preferably R² represents a hydrogen atom.

**5.** A compound according to one or more of claims 1 to 4, **characterised in that** R³ represents a saturated or unsaturated, optionally at least mono-substituted 3- to 9-membered cycloaliphatic radical which may contain 1, 2 or 3 heteroatom(s) independently selected from the group consisting of nitrogen, oxygen and sulphur as ring member(s) and which may be condensed with an optionally at least mono-substituted mono- or bicyclic ring system,
whereby the rings of the ring system are 5- 6- or 7-membered and may contain 1, 2 or 3 heteroatom(s) independently selected from the group consisting of nitrogen, oxygen and sulphur;
or R³ represents a ―NR¹³R¹⁴ moiety;
preferably R³ represents a moiety selected from the group consisting of 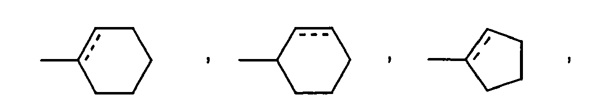 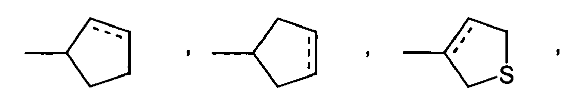 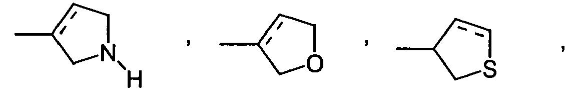 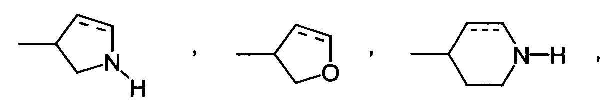 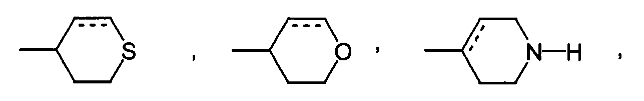 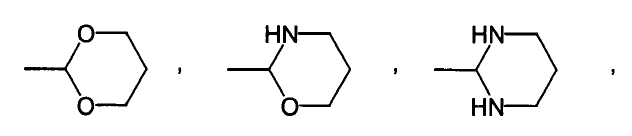 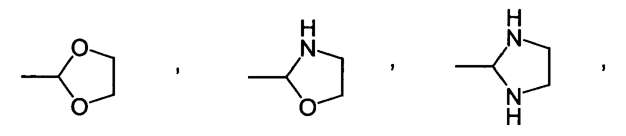 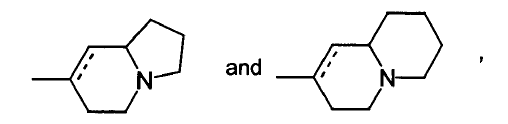 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅₋alkyl, -O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the -NH groups; and
if present, the dotted line represents an optional chemical bond;
or R³ represents a ―NR¹³R¹⁴ moiety;
more preferably R³ represents a ―NR¹³R¹⁴ moiety.

**6.** A compound according to one or more of claims 1 to 5, **characterised in that** R⁴, R⁵, R⁶ and R⁷, identical or different, each represent a hydrogen atom; ― NO₂; -NH₂; -SH; -OH; -CN; -C(=O)-OH; -C(=O)-H; -S(=O)₂-OH; -C(=O)-NH₂; - S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, -NR¹⁸R¹⁹; -C(=O)-NHR²⁰, -C(=O)-NR²¹R²²;-S(=O)₂₋NHR²³, -S(=O)₂-NR²⁴R²⁵; -O-C(=O)-R²⁶; -NH-C(=O)-R²⁷; -NR²⁸-C(=O)-R²⁹; NH-C(=O)-O-R³⁰; NR³¹-C(=O)-O-R³²; -S(=O)₂-O-R³³; F, Cl, Br, l; -CF₃, -CHF₂, -CH₂F, a linear or branched, unsubstituted C₁₋₁₀ alkyl radical; a linear or branched, unsubstituted C₂₋₆ alkenyl radical; a linear or branched, unsubstituted C₂₋₆ alkinyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatom(s) independently selected from the group consisting of nitrogen, oxygen and sulphur as ring member(s); or an optionally at least mono-substituted 5- to 10-membered aryl or heteroaryl radical, which may be bonded via a linear or branched C₁₋₆-alkylene group and wherein the heteroaryl radical contains 1, 2 or 3 heteroatom(s) independently selected from the group consisting of nitrogen, oxygen and sulphur as ring member(s);
preferably R⁴, R⁵, R⁶ and R⁷, identical or different, each represent a hydrogen atom; ―NO₂; -NH₂; -SH; -OH; -CN; -C(=O)-OH; -C(=O)-H; -S(=O)₂-OH; -C(=O)-NH₂; -S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, -NR¹⁸R¹⁹; -C(=O)-NHR²⁰, -C(=O)-NR²¹R²²; -S(=O)₂₋NHR²³, -S(=O)₂-NR²⁴R²⁵; -O-C(=O)-R²⁶; -NH-C(=O)-R²⁷; -NR²⁸-C(=O)-R²⁹; NH-C(=O)-O-R³⁰ ; NR³¹-C(=O)-O-R³²; -S(=O)₂-O-R³³ ; F, Cl, Br, l; -CF₃, -CHF₂, -CH₂F, an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -0-C(=O)-C₁₋₅-alkyl, F, CI, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ― (CH₂)₁, ₂ or ₃- group and/or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-O-C₁₋₅-alkyl, -O-C(=O)-C₁₋₅-alkyl, F, Cl, Br, l, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl;
more preferably R⁴, R⁵, R⁶ and R⁷, identical or different, each represent a hydrogen atom; ―NO₂; -NH₂; -SH; -OH; -CN; -C(=O)-OH; -C(=O)-H; -S(=O)₂₋OH; -C(=O)-NH₂; -S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷ -NR¹⁸R¹⁹. F, Cl, Br, I; -CF₃, -CHF₂, -CH₂F, an unsubstituted alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, - O-C₁₋₅-alkyl, -S-C₁₋₅-alkyl, -C(=O)-O-C₁₋₅-alkyl, -O-C(=O)-C₁₋₅-alkyl, F, Cl, Br, l, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
in each case with the proviso that at least one of the substituents R⁴, R⁵, R⁶ and R⁷ represents an -N(R¹⁵)-S(=O)₂-R¹⁶ moiety and at least one of the further substituents R⁴, R⁵, R⁶ and R⁷ does not represent hydrogen.

**7.** A compound according to one or more of claims 1 to 6, **characterised in that** one of the substituents R⁴, R⁵, R⁶ and R⁷ represents an -N(R¹⁵)-S(=O)₂-R¹⁶ moiety and at least one of the further substituents R⁴, R⁵, R⁶ and R⁷ does not represent a hydrogen atom;
preferably one of the substituents R⁴, R⁵, R⁶ and R⁷ represents an -N(R¹⁵)-S(=O)₂-R¹⁶ moiety and one of the further substituents R⁴, R⁵, R⁶ and R⁷ does not represent a hydrogen atom.

**8.** A compound according to one or more claims 1 to 7, **characterized in that** R⁴ represents an -N(R¹⁵)-S(=O)₂-R¹⁶ moiety and at least one of the substituents R⁵, R⁶ and R⁷ does not represent a hydrogen atom;
preferably R⁴ represents an -N(R¹⁵)-S(=O)₂-R¹⁶ moiety and one of the substituents R⁵, R⁶ and R⁷ does not represent a hydrogen atom.

**9.** A compound according to one or more of claims 1 to 7, **characterized in that** R⁵ represents an -N(R¹⁵)-S(=O)₂-R¹⁶ moiety and at least one of the substituents R⁴, R⁶ and R⁷ does not represent a hydrogen atom;
preferably R⁵ represents an -N(R¹⁵)-S(=O)₂-R¹⁶ moiety and one of the substituents R⁴, R⁶ and R⁷ does not represent a hydrogen atom.

**10.** A compound according to one or more of claims 1 to 7, **characterised in that** R⁶ represents an -N(R¹⁵)-S(=O)₂-R¹⁶ moiety and at least one of the substituents R⁴, R⁵ and R⁷ does not represent a hydrogen atom;
preferably R⁶ represents an -N(R¹⁵)-S(=O)₂-R¹⁶ moiety and one of the substituents R⁴, R⁵ and R⁷ does not represent a hydrogen atom.

**11.** A compound according to one or more of claims 1 to 7, **characterised in that** R⁷ represents an -N(R¹⁵)-S(=O)₂-R¹⁶ moiety and at least one of the substituents R⁴, R⁵ and R⁶ does not represent a hydrogen atom;
preferably R⁷ represents an -N(R¹⁵)-S(=O)₂-R¹⁶ moiety and one of the substituents R⁴, R⁵ and R⁶ does not represent a hydrogen atom.

**12.** A compound according to one or more of claims 1 to 11, **characterized in that** R⁸ represents a hydrogen atom or a linear or branched C₁₋₁₀ alkyl radical, preferably represents a hydrogen atom or an alkyl radical selected from the group consisting of methyl, ethyl, n-propyl, iso-propyl, n-butyl, sec-butyl, iso-butyl and tert-butyl.

**13.** A compound according to one or more of claims 1 to 12, **characterised in that** R¹², R¹⁷, R¹⁸, R¹⁹, R²⁰, R²¹, R²², R²³, R²⁴, R²⁵, R²⁶, R²⁷, R²⁸, R²⁹, R³⁰, R³¹, R³² and R³³, independent from one another, each represent a linear or branched, unsubstituted C₁₋₁₀ alkyl radical; a linear or branched, unsubstituted C₂₋₆ alkenyl radical; a linear or branched, unsubstituted C₂₋₆alkinyl radical; a saturated or unsaturated, optionally at least mono-substituted, 3- to 9-membered cycloaliphatic radical, which may be bonded via a linear or branched C₁₋₆-alkylene group and/or which may contain 1, 2 or 3 heteroatom(s) independently selected from the group consisting of nitrogen, oxygen and sulphur as ring member(s); or an optionally at least mono-substituted 5- to 10-membered aryl or heteroaryl radical, which may be bonded via a linear or branched C₁₋₆-alkylene, C₂₋₆-alkenylene or C₂₋₆₋alkinylene group and wherein the heteroaryl radical contains 1, 2 or 3 heteroatom(s) independently selected from the group consisting of nitrogen, oxygen and sulphur as ring member(s);
preferably R¹² R¹⁷, R¹⁸, R¹⁹, _{R}²⁰, R²¹, R²², R²³, R²⁴, R²⁵, R²⁶, R²⁷, R²⁸, R²⁹, R³⁰, R³¹, R³² and R³³, independent from one another, each represent an unsubstituted alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -0-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a moiety selected from the group consisting of ―(CH₂)-, -(CH₂)₂-, - (CH₂)₃- and ―(CH=CH)- and/or may be substituted with 1, 2, 3, 4 or 5 substituent(s) independently selected from the group consisting of C₁₋₅-alkyl, - O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

**14.** A compound according to one or more of claims 1 to 13, **characterised in that** R⁹ represents an optionally at least mono-substituted 5- to 10-membered aryl or heteroaryl radical, which may be bonded via a linear or branched C₁₋₆ alkylene, C₂₋₆ alkenylene or C₂₋₆ alkinylene group and wherein the heteroaryl radical contains 1, 2 or 3 heteroatom(s) independently selected from the group consisting of nitrogen, oxygen and sulphur as ring member(s) and which may be condensed with an optionally at least mono-substituted mono- or bicyclic ring system;
whereby the rings of the ring system are 5- 6- or 7-membered and may contain 1, 2 or 3 heteroatom(s) independently selected from the group consisting of nitrogen, oxygen and sulphur,
preferably R⁹ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)2-C₁₋₅-alkyl, -S(=O)₂₋phenyl, cyclopropyl, cyclobutyl, cyclopentyl, cyclohexyl, phenyl, phenoxy and benzyl.

**15.** A compound according to one or more of claims 1 to 14, **characterised in that** R¹⁰ and R¹¹, independent from one another, each represent a linear or branched, unsubstituted C₁₋₁₀ alkyl radical; a linear or branched, unsubstituted C₂₋₆ alkenyl radical; a linear or branched, unsubstituted C₂₋₆ alkinyl radical; or an optionally at least mono-substituted 5- to 10- membered aryl or heteroaryl radical, which may be bonded via a linear or branched C₁₋₆-alkylene group and wherein the heteroaryl radical contains 1, 2 or 3 heteroatom(s) independently selected from the group consisting of nitrogen, oxygen and sulphur as ring member(s);
preferably R¹⁰ and R¹¹, independent from one another, each represent an unsubstituted alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, - O-C₁₋₅-alkyl, -S-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, - NO₂, -CHO, -CF₂H, -CFH₂, -C(=O)-NH₂, -C(=O)-NH(C₁₋₅-alkyl), -C(=O)-N(C₁₋₅₋alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

**16.** A compound according to one or more of claims 1 to 15, **characterised in that** R¹³ and R¹⁴, independent from one another, each represent a hydrogen atom; a linear or branched, unsubstituted C₁₋₁₀ alkyl radical; a linear or branched, unsubstituted C₂₋₆ alkenyl radical; or a linear or branched, unsubstituted C₂₋₆ alkinyl radical;
or R¹³ and R¹⁴ together with the bridging nitrogen form an optionally at least mono-substituted, saturated or unsaturated, but not aromatic 3- to 9-membered heterocyclic ring which may contain 1, 2 or 3 heteroatom(s) independently selected from the group consisting of nitrogen, oxygen and sulphur as ring member(s) and/or which may be condensed with an optionally at least mono-substituted mono- or bicyclic ring system,
whereby the rings of the ring system are 5- 6- or 7-membered and may contain 1, 2 or 3 heteroatom(s) independently selected from the group consisting of nitrogen, oxygen and sulphur;
preferably R¹³ and R¹⁴, independent from one another, each represent a hydrogen atom; or an unsubstituted alkyl radical selected from the group consisting of methyl, ethyl, n-propyl, iso-propyl, n-butyl, sec-butyl, iso-butyl and tert-butyl; or
R¹³ and R¹⁴ together with the bridging nitrogen atom form a moiety selected from the group consisting of 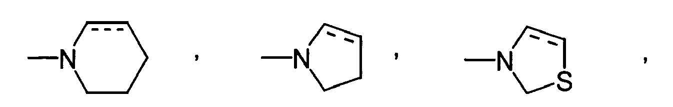 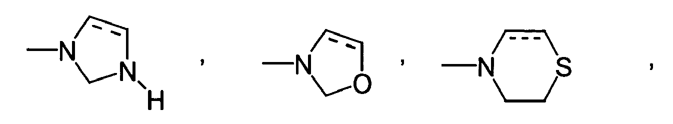 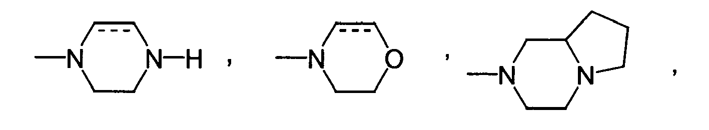 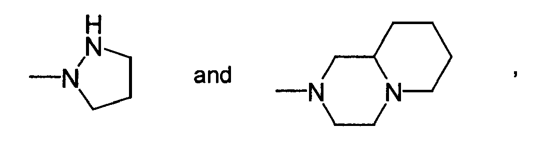 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅₋alkyl, -O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the ―NH groups; and
if present, the dotted line represents an optional chemical bond.

**17.** A compound according to one or more of claims 1 to 16, **characterised in that** R¹⁵ represents a hydrogen atom; a linear or branched, unsubstituted C₁₋₁₀ alkyl radical; a linear or branched, unsubstituted C₂₋₆ alkenyl radical; a linear or branched, unsubstituted C₂₋₆ alkinyl radical or a ―S(=O)₂-R¹⁶ moiety;
preferably R¹⁵ represents a hydrogen atom; an unsubstituted alkyl radical selected from the group consisting of methyl, ethyl, n-propyl, iso-propyl, n-butyl, sec-butyl, iso-butyl and tert-butyl or a ―S(=O)₂-R¹⁶ moiety;
more preferably R¹⁵ represents a hydrogen atom.

**18.** A compound according to one or more of claims 1 to 17, **characterised in that** R¹⁶ represents a linear or branched, unsubstituted C₁₋₁₀ alkyl radical; a linear or branched, unsubstituted C₂₋₆ alkenyl radical; a linear or branched, unsubstituted C₂₋₆ alkinyl radical; a saturated or unsaturated, optionally at least mono-substituted 3- to 9-membered cycloaliphatic radical, which may be bonded via a linear or branched C₁₋₆ alkylene, C₂₋₆ alkenylene or C₂₋₆ alkinylene group and/or which may contain 1, 2 or 3 heteroatom(s) independently selected from the group consisting of nitrogen, oxygen and sulphur as ring member(s); an optionally at least mono-substituted 5- to 10-membered aryl or heteroaryl radical, which may be bonded via a linear or branched C₁₋₆ alkylene, C₂₋₆ alkenylene or C₂₋₆ alkinylene group and wherein the heteroaryl radical contains 1, 2 or 3 heteroatom(s) independently selected from the group consisting of nitrogen, oxygen and sulphur as ring member(s) and which may be condensed with an optionally at least mono-substituted mono- or bicyclic ring system;
whereby the rings of the ring system are 5- 6- or 7-membered and may contain 1, 2 or 3 heteroatom(s) independently selected from the group consisting of nitrogen, oxygen and sulphur,
preferably R¹⁶ represents an unsubstituted alkyl radical selected from the group consisting of methyl, ethyl, n-propyl, iso-propyl, n-butyl, sec-butyl, iso-butyl and tert-butyl;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via moiety selected from the group consisting of ―(CH₂)-, - (CH₂)₂-, -(CH₂)₃- and ―(CH=CH)- and/or may be substituted with 1, 2, 3, 4 or 5 substituent(s) independently selected from the group consisting of C₁₋₅-alkyl, - O-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, l, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ― (CH₂)_{1,2 or 3}- group and/or may be substituted with 1, 2, 3, 4 or 5 substituent(s) independently selected from the group consisting of C₁₋₅-alkyl, -O-C₁₋₅-alkyl, - S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl.

**19.** A compound according to one or more of claims 1 to 18, **characterised in that** n is 0, 1 or 2; preferably n is 0 or 1, more preferably n is 0.

**20.** A compound according to one or more of claims 1 to 19, **characterised in that**
n is 0;
R¹ represents a hydrogen atom;
R² represents a hydrogen atom;
R³ represents a ―NR¹³R¹⁴ moiety;
R⁴, R⁵, R⁶ and R⁷, identical or different, each represent a hydrogen atom; ― NO₂; -NH₂; -SH; -OH; -CN; -C(=O)-OH; -C(=O)-H; -S(=O)₂-OH; -C(=O)-NH₂; - S(=O)₂-NH₂; -C(=O)-R⁸; -S(=O)₂-R⁹; -OR¹⁰; -SR¹¹; -C(=O)-OR¹²; -N(R¹⁵)-S(=O)₂-R¹⁶; -NH-R¹⁷, -NR¹⁸R¹⁹; F, Cl, Br, l; -CF₃, -CHF₂, -CH₂F, an unsubstituted alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ― (CH₂)_{1,2 or 3}- group and/or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl;
with the proviso that at least one of the substituents R⁴, R⁵, R⁶ and R⁷ represents an -N(R¹⁵)-S(=O)₂-R¹⁶ moiety and at least one of the other substituents of R⁴, R⁵, R⁶ and R⁷ does not represent hydrogen,
R⁸, R¹² and R¹⁷ to R¹⁹, independent from one another, each represent an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via a ―(CH₂)_{1,2 or 3}- group and/or may be substituted with 1, 2, 3, 4 or 5 substituent(s) independently selected from the group consisting of C₁₋₅₋alkyl, -O-C₁₋₅-alkyl, -S-C₁₋₅-alkyl, oxo (=O), thia (=S), -C(=O)-O-C₁₋₅-alkyl, -0-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a moiety selected from the group consisting of ―(CH₂)-, -(CH₂)₂-, -(CH₂)₃- and ― (CH=CH)- and/or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl;
R⁹ represents an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ― (CH₂)_{1,2 or 3}- group and/or may be substituted with 1, 2, 3, 4 or 5 substituent(s) independently selected from the group consisting of C₁₋₅-alkyl, -O-C₁₋₅-alkyl, - S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl;
R¹⁰ and R¹¹, independent from one another, each represent an unsubstituted alkyl radical selected from the group consisting of methyl, ethyl, n-propyl, iso-propyl, n-butyl, sec-butyl, iso-butyl and tert-butyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl, whereby said aryl or heteroaryl radical may be bonded via a ―(CH₂)₁, 2 or 3-group and/or may be substituted with 1, 2, 3, 4 or 5 substituent(s) independently selected from the group consisting of C₁₋₅-alkyl, -O-C₁₋₅-alkyl, - S-C₁₋₅-alkyl, -C(=O)-O-C₁₋₅-alkyl, -O-C(=O)-C₁₋₅-alkyl, F, CI, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, cyclopropyl, cyclobutyl, cyclopentyl, cyclohexyl, phenyl, phenoxy and benzyl;
R¹³ and R¹⁴, independent from one another, each represent a hydrogen atom; or an unsubstituted alkyl radical selected from the group consisting of methyl, ethyl, n-propyl, iso-propyl, n-butyl, sec-butyl, iso-butyl and tert-butyl;
or
R¹³ and R¹⁴ together with the bridging nitrogen atom form a moiety selected from the group consisting of 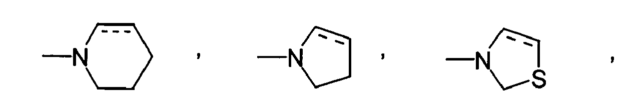 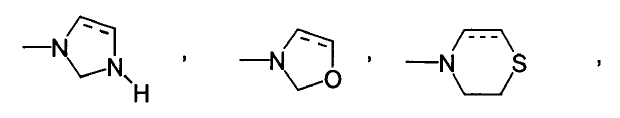 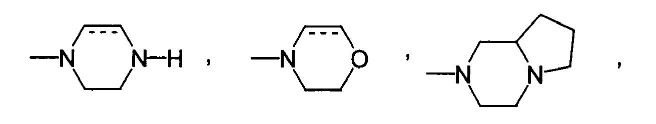 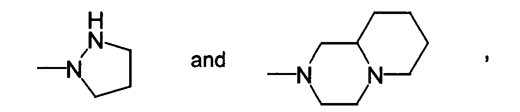 whereby each of these afore mentioned cyclic moieties may be substituted with 1, 2 or 3 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅₋alkyl, -O-C(=O)-C₁₋₅-alkyl, F, Cl, Br, I, -CN, -CF₃, -OCF₃, -SCF₃, -OH, -SH, - NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, cyclopropyl, cyclobutyl, cyclopentyl, cyclohexyl, phenyl, phenoxy and benzyl in any position including the NH-groups; and, if present, the dotted line represents an optional chemical bond;
R¹⁵ represents a hydrogen atom
and
R¹⁶ represents an unsubstituted alkyl radical selected from the group consisting of methyl, ethyl, n-propyl, iso-propyl, n-butyl, sec-butyl, iso-butyl and tert-butyl; a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl and azepanyl, whereby said (hetero)cycloaliphatic radical may be bonded via moiety selected from the group consisting of ―(CH₂)-, -(CH₂)₂-, -(CH₂)₃- and ―(CH=CH)- and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), - C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, - C(=O)-NH₂, -C(=O)-NH(C₁₋₅-alkyl), -C(=O)-N(C₁₋₅-alkyl)₂, -S(=O)₂-C₁₋₅-alky), - S(=O)₂-phenyl, cyclopropyl, cyclobutyl, cyclopentyl, cyclohexyl, phenyl, phenoxy and benzyl; or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, tetrazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl, imidazo[2,1-b]thiazolyl, indanyl, indenyl, tetrahydronaphthyl, tetrahydroisoquinolinyl, benzodioxolyl, benzodioxanyl, tetrahydrocarbazolyl, 2,3-dihydrobenzo[d]thiazolyl, benzimidazolidinyl, chromenyl, isochromanyl and chromanyl, whereby said aryl or heteroaryl radical may be bonded via a ―(CH₂)_{1,2 or 3}- group and/or may be substituted with 1, 2, 3, 4 or 5 substituent(s) independently selected from the group consisting of C₁₋₅-alkyl, -O-C₁₋₅-alkyl, -S-C₁₋₅-alkyl, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, cyclopropyl, cyclobutyl, cyclopentyl, cyclohexyl, phenyl, phenoxy and benzyl;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

**21.** Process for the preparation of a compound according to one or more of claims 1 to 20, **characterised in that** at least one compound of general formula ll, 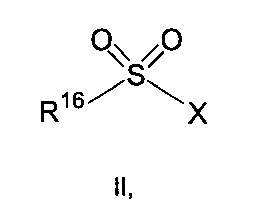
wherein R¹⁶ has the meaning according to one or more of claims 1 to 20 and X represents a leaving group, preferably a halogen atom, particularly preferably a chlorine atom, is reacted with at least one compound of general formula III, 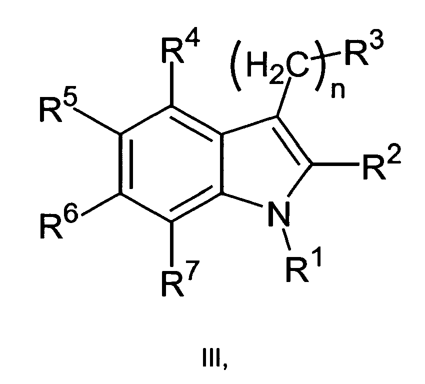
wherein R¹ to R⁷ and n have the meaning according to one or more of claims 1 to 20, with the proviso that at least one substituent of the group consisting of R⁴, R⁵, R⁶ and R⁷ represents a ―N(H)(R¹⁵) moiety or a protected derivative thereof and at least one of the further substituents R⁴, R⁵, R⁶ and R⁷ does not represent hydrogen, in a suitable reaction medium, preferably in the presence of at least one base.

**22.** Medicament comprising at least one substituted indole compound according to one or more of claims 1 to 20, and optionally at least one physiologically acceptable auxiliary agent.

**23.** Medicament according to claim 22 for the prophylaxis and/or treatment of a disorder or a disease that is at least partially mediated via 5-HT₆-receptors.

**24.** Medicament according to claim 22 or 23 for the regulation of appetite, for the maintenance, increase or reduction of body weight, for the prophylaxis and/or treatment of a disorder or a disease related to food intake, preferably for the prophylaxis and/or treatment of obesity, bulimia, anorexia, cachexia or type II diabetes (non insulin dependent diabetes mellitus); more preferably for the prophylaxis and/or treatment of obesity.

**24.** Medicament according to claim 22 or 23 for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; or hyperactivity disorder (ADHD, attention deficit/hyperactivity disorder) or for the improvement of cognition (cognitive enhancement), preferably for the improvement of cognition (cognitive enhancement).

**25.** Use of at least one substituted indole compound according to one or more of claims 1 to 20 for the manufacture of a medicament for the prophylaxis and/or treatment of a disorder or a disease that is at least partially mediated via 5-HT₆-receptors.

**26.** Use of at least one substituted indole compound according to one or more of claims 1 to 20 for the manufacture of a medicament for the regulation of appetite, for the maintenance, increase or reduction of body weight, for the prophylaxis and/or treatment of a disorder or a disease related to food intake, preferably for the prophylaxis and/or treatment of obesity, bulimia, anorexia, cachexia or type II diabetes (non insulin dependent diabetes mellitus); more preferably for the prophylaxis and/or treatment of obesity.

**26.** Use of at least one substituted indole compound according to one or more of claims 1 to 20 for the manufacture of a medicament for the prophylaxis and/or treatment of irritable colon syndrome; disorders of the central nervous system; anxiety; panic attacks; depression; bipolar disorders; cognitive disorders; memory disorders; senile dementia; psychosis; neurodegenerative disorders, preferably selected from the group consisting of Morbus Alzheimer, Morbus Parkinson, Morbus Huntington and Multiple Sclerosis; schizophrenia; psychosis; or hyperactivity disorder (ADHD, attention deficit/hyperactivity disorder) or for the improvement of cognition (cognitive enhancement), preferably for the improvement of cognition (cognitive enhancement).
